# EUROPEAN PATENT APPLICATION

(11) **EP 4 417 118 A1**
(43) Date of publication of application: **21.08.2024**
(21) Application number: 22880434.0
(22) Date of filing: 14.10.2022
(51) Int. Cl.: A61B 5/03, A61B 1/015

(54) **PERFUSION SYSTEM FOR ENDOSCOPE SYSTEM, AND WORKING METHOD AND ELECTRONIC DEVICE**

(30) Priority: 15.10.2021 CN 202111205657
(71) Applicant: Shenzhen Mindray Bio-Medical Electronics Co., Ltd., Shenzhen, Guangdong 518057 (CN)
(72) Inventor: YANG, Taorui, Shenzhen, Guangdong 518057 (CN); PENG, Mingxu, Shenzhen, Guangdong 518057 (CN); ZUO, Pengfei, Shenzhen, Guangdong 518057 (CN)
(74) Representative: KIPA AB
(86) International application number: PCT/CN2022/125487
(87) International publication number: WO 2023/061498

(57) **Abstract**

A perfusion system for an endoscopy system (90), and a working method and an electronic device. The method comprises: in response to the input of an enablement command, acquiring at least one group of state parameters of a perfusion pipeline (10), and according to the state parameters, acquiring an association relationship, wherein the enablement command is used for enabling a surgical function, the surgical function comprises a perfusion function, an exhaust function, a flushing function and/or a cavity expansion function, and the association relationship is used for indicating the relationship between a pressure value, which is measured by a pressure sensor (30), and a pressure value of an outflow end of an endoscopy system (90).

## Description

### TECHNICAL FIELD

The disclosure relates to a perfusion system for an endoscope system, and a working method and an electronic device.

### BACKGROUND

A perfusion system is mainly used for surgery inside body cavity, etc. One end of the perfusion system is connected with a medium container, and the other end of the perfusion system is connected with an endoscope system. A wide and clear field of view can be obtained by transporting a medium inside the medium container into a body cavity to be observed, so as to distend the body cavity, so that the doctor can conduct subsequent surgical functions. Therefore, the perfusion system is widely used in intracavitary surgeries for such as kidney and ureter, as well as, in surgeries inside a uterine cavity .

Now take the uterine cavity as an example. In this case, the endoscope system is also called a hysteroscopic system. When performing a uterine cavity-related surgery, the perfusion system is configured to connect a liquid bag containing a uterine distension fluid and the hysteroscopic system. A uterine distension pump of the perfusion system squeezes a perfusion pipeline to allow the uterine distension fluid to enter into a pipeline of the hysteroscopic system from the liquid bag, and further enter into the uterine cavity through the pipeline of the hysteroscopic system, thereby distending the uterine cavity for obtaining a wide and clear field of view, which is convenient for a doctor to observe lesions and perform surgery. During the surgery, a calculation accuracy of intrauterine pressure affects a progress of the surgery and a life safety of a patient. When a uterine distension pressure is too low, the uterine distension effect is unsatisfactory, the field of vision is unclear, and the perfusion is not able to quickly remove impurities in the cavity. When the uterine distension pressure is too high, an intracavitary wound continues to absorb the uterine distension fluid, and in severe cases causes the patient to suffer from "water intoxication" and even endangers the life of the patient. In order to ensure the safety of the patient, during surgery, the uterine distension pump is required to accurately calculate a pressure inside the uterine cavity and control the pressure inside the uterine cavity within a certain range preset by the doctor.

Therefore, when using a perfusion system to perfuse a cavity, how to accurately obtain the pressure inside the cavity is an issue worth studying.

### SUMMARY

This disclosure mainly provides a perfusion system for an endoscope system, and a working method and an electronic device, which are described in detail below.

In an embodiment, a perfusion system for an endoscope system is provided, including:
a perfusion pipeline, wherein one end of the perfusion pipeline is connected with a medium container, and the other end of the perfusion pipeline is connected with the endoscope system;
a power pump mechanism, which is configured to drive a medium inside the medium container to flow into the endoscope system through the perfusion pipeline;
a pressure sensor, which is configured to measure a pressure value inside the perfusion pipeline; and
a processor, which is configured to obtain, in response to an input of a start instruction, one group of state parameters of the perfusion pipeline, and to obtain another group of state parameters of the perfusion pipeline after adjusting a pump working parameter of the power pump mechanism, wherein each group of state parameters include a pressure value which is obtained by the pressure sensor, and a flow value which is obtained by the pump working parameter of the power pump mechanism and/or by a flow sensor which is arranged at the perfusion pipeline; wherein the start instruction is configured to start a surgical function which includes a perfusion function, an exhaust function, a flushing function and/or a cavity distending function;
the processor is further configured to calculate an actual flow-resistance relationship according to the at least two groups of state parameters, calculate a similarity between the actual flow-resistance relationship and each sample flow-resistance relationship in a preset database, and obtain a target flow-resistance relationship according to the similarity; or the processor is further configured to fit to obtain a target flow-resistance relationship, which corresponds to a currently used endoscope system, according to the at least two groups of state parameters;
the processor is further configured to obtain a real-time pump working parameter of the power pump mechanism, and calculate a resistance according to the real-time pump working parameter of the power pump mechanism and the target flow-resistance relationship; and to adjust and/or calculate a real-time pressure value of an outflow end of the endoscope system according to a real-time pressure value of the pressure sensor and the calculated resistance.

In an embodiment, a perfusion system for an endoscope system is provided, including:
a perfusion pipeline, wherein one end of the perfusion pipeline is connected with a medium container, and the other end of the perfusion pipeline is connected with the endoscope system;
a power pump mechanism, which is configured to drive a medium inside the medium container to flow into the endoscope system through the perfusion pipeline;
a pressure sensor, which is configured to measure a pressure value inside the perfusion pipeline; and
a processor, which is configured to obtain, in response to an input of a start instruction, at least one group of state parameters of the perfusion pipeline, and to obtain an association relationship according to the state parameters; wherein the start instruction is configured to start a surgical function which includes a perfusion function, an exhaust function, a flushing function and/or a cavity distending function; wherein the association relationship is configured to indicate a relationship between a pressure value measured by the pressure sensor and a pressure value of an outflow end of the endoscope system.

In an embodiment, a perfusion system for an endoscope system is provided, including:
a perfusion pipeline, wherein one end of the perfusion pipeline is connected with a medium container, and the other end of the perfusion pipeline is connected with the endoscope system;
a power pump mechanism, which is configured to drive a medium inside the medium container to flow into the endoscope system through the perfusion pipeline;
a pressure sensor, which is configured to measure a pressure value inside the perfusion pipeline; and
a processor, which is configured to obtain, in response to an input of a start instruction, at least one group of state parameters of the perfusion pipeline, and to obtain an association relationship according to the state parameters; wherein the start instruction is configured to start a surgical function, which is configured to enable the medium inside the perfusion pipeline to flow, so as to enter into a preset cavity of a living being through the perfusion pipeline and the endoscope system for performing surgery; wherein the association relationship is configured to indicate a relationship between a pressure value measured by the pressure sensor and a pressure value of an outflow end of the endoscope system.

In an embodiment, a perfusion system for an endoscope system is provided, including:
a perfusion pipeline, wherein one end of the perfusion pipeline is connected with a medium container, and the other end of the perfusion pipeline is connected with the endoscope system;
a power pump mechanism, which is configured to drive a medium inside the medium container to flow into the endoscope system through the perfusion pipeline;
a pressure sensor, which is configured to measure a pressure value inside the perfusion pipeline;
a memory, which is configured to store execution program(s) of a perfusion process of the perfusion system, wherein the perfusion process is at least divided into a first phase and a second phase;
a processor, which is configured to enable, in response to an input of a start instruction, the perfusion process to continuously enter into the first phase and the second phase;
wherein the start instruction is configured to start a surgical function which includes a perfusion function, an exhaust function, a flushing function and/or a cavity distending function; in the first phase, the processor is further configured to obtain an association relationship, wherein the association relationship is configured to indicate a relationship between a pressure value measured by the pressure sensor and a pressure value of an outflow end of the endoscope system; in the second phase, the processor is further configured to use the association relationship to perform the surgical function.

In an embodiment, a perfusion system for an endoscope system is provided, including:
a perfusion pipeline, wherein one end of the perfusion pipeline is connected with a medium container, and the other end of the perfusion pipeline is connected with the endoscope system;
a power pump mechanism, which is configured to drive a medium inside the medium container to flow into the endoscope system through the perfusion pipeline;
a pressure sensor, which is configured to measure a pressure value inside the perfusion pipeline;
a memory, which is configured to store execution program(s) of a perfusion process of the perfusion system, wherein the perfusion process is at least divided into a first phase and a second phase;
a processor, which is configured to enable, in response to an input of a start instruction, the perfusion process to continuously enter into the first phase and the second phase;
wherein the start instruction is configured to start a surgical function which includes a perfusion function, an exhaust function, a flushing function and/or a cavity distending function; in the first phase, the processor is further configured to obtain at least one group of state parameters of the perfusion pipeline, wherein each group of state parameters include a pressure value which is obtained by the pressure sensor, and a flow value which is obtained by the pump working parameter of the power pump mechanism and/or by a flow sensor which is arranged at the perfusion pipeline; in the second phase, the processor is further configured to perform the surgical function according to the state parameters.

In an embodiment, in order to perform the surgical function according to the state parameters, the processor is further configured to:
obtain an association relationship according to the state parameters; wherein the association relationship is configured to indicate a relationship between a pressure value measured by the pressure sensor and a pressure value of an outflow end of the endoscope system; and
use the association relationship to perform the surgical function.

In an embodiment, a perfusion system for an endoscope system is provided, including:
a perfusion pipeline, wherein one end of the perfusion pipeline is connected with a medium container, and the other end of the perfusion pipeline is connected with the endoscope system;
a power pump mechanism, which is configured to drive a medium inside the medium container to flow into the endoscope system through the perfusion pipeline;
a pressure sensor, which is configured to measure a pressure value inside the perfusion pipeline;
a memory, which is configured to store execution program(s) of a perfusion process of the perfusion system, so as to perform a first function and a second function;
a processor, which is configured to automatically perform, in response to an input of a start instruction, the first function and the second function; in order to perform the first function, the processor is further configured to obtain an association relationship, wherein the association relationship is configured to indicate a relationship between a pressure value measured by the pressure sensor and a pressure value of an outflow end of the endoscope system; in order to perform the second function, the processor is further configured to use the association relationship to perform a surgical function;
wherein the surgical function includes a perfusion function, an exhaust function, a flushing function and/or a cavity distending function.

In an embodiment, a perfusion system for an endoscope system is provided, including:
a perfusion pipeline, wherein one end of the perfusion pipeline is connected with a medium container, and the other end of the perfusion pipeline is connected with the endoscope system;
a power pump mechanism, which is configured to drive a medium inside the medium container to flow into the endoscope system through the perfusion pipeline;
a pressure sensor, which is configured to measure a pressure value inside the perfusion pipeline; and
a processor, which is configured to obtain, in response to an input of a start instruction, at least one group of state parameters of the perfusion pipeline, and to obtain an association relationship according to the state parameters; wherein the start instruction is configured to start a surgical function which includes a perfusion function, an exhaust function, a flushing function and/or a cavity distending function; wherein the association relationship is configured to indicate a relationship between a pressure value measured by the pressure sensor and a pressure value of an outflow end of the endoscope system;
wherein after obtaining the association relationship, the processor is further configured to re-obtain at least one group of state parameters of the perfusion pipeline and update the association relationship according to the re-obtained state parameters, when determining that the endoscope system is replaced.

In an embodiment, in order to obtain an association relationship, the processor is further configured to:
obtain one group of state parameters of the perfusion pipeline, and obtain another group of state parameters of the perfusion pipeline after adjusting a pump working parameter of the power pump mechanism, wherein each group of state parameters include a pressure value which is obtained by the pressure sensor, and a flow value which is obtained by the pump working parameter of the power pump mechanism and/or by a flow sensor which is arranged at the perfusion pipeline; and
obtain the association relationship according to the at least two groups of state parameters.

In an embodiment, in order to obtain one group of state parameters of the perfusion pipeline, and obtain another group of state parameters of the perfusion pipeline after adjusting a pump working parameter of the power pump mechanism, the processor is further configured to:
adjust a pump working parameter of the power pump mechanism, so as to enable a pressure value of the pressure sensor to be Fn, obtain a current pump working parameter Rn of the power pump mechanism, and take Fn and Rn as the one group of state parameters; and adjust a pump working parameter of the power pump mechanism, so as to enable a pressure value of the pressure sensor to be Fn+1, obtain a current pump working parameter Rn+1 of the power pump mechanism, and take Fn+1 and Rn+1 as the another group of state parameters; or
adjust a pump working parameter of the power pump mechanism to be R'n, so as to control the power pump mechanism to work, obtain a pressure value F'n of the pressure sensor when said pump working parameter of the power pump mechanism is R'n, and take F'n and R'n as the one group of state parameters; and adjust a pump working parameter of the power pump mechanism to be R'n+1, so as to control the power pump mechanism to work, obtain a pressure value F'n+1 of the pressure sensor when said pump working parameter of the power pump mechanism is R'n+1, and take F'n+1 and R'n+1 as the another group of state parameters.

In an embodiment, in order to obtain an association relationship, the processor is further configured to:
obtain one group of state parameters of the perfusion pipeline, wherein the state parameters include a pressure value which is obtained by the pressure sensor, and a flow value which is obtained by the pump working parameter of the power pump mechanism and/or by a flow sensor which is arranged at the perfusion pipeline; and
obtain the association relationship according to said one group of state parameters.

In an embodiment, in order to obtain an association relationship, the processor is further configured to:
select from a preset database, in which one or more sample flow-resistance relationships are preset, according to at least one group of state parameters, one sample flow-resistance relationship as a target flow-resistance relationship; wherein the target flow-resistance relationship is configured to indicate difference(s) between pressure value(s) measured by the pressure sensor and pressure value(s) at the outflow end of the endoscope system at different flow rates; and
use the target flow-resistance relationship as the association relationship.

In an embodiment, in order to select from a preset database, in which one or more sample flow-resistance relationships are preset, according to the at least one group of state parameters, one sample flow-resistance relationship as a target flow-resistance relationship, the processor is further configured to:
calculate an actual flow-resistance relationship according to the at least one group of state parameters, then calculate a similarity between the actual flow-resistance relationship and each sample flow-resistance relationship in the preset database;
select a sample flow-resistance relationship which corresponds to a maximum similarity as the target flow-resistance relationship, if the maximum similarity is greater than a first similarity threshold.

In an embodiment, in order to select from a preset database, in which one or more sample flow-resistance relationships are preset, according to the at least one group of state parameters, one sample flow-resistance relationship as a target flow-resistance relationship, the processor is further configured to:
calculate an actual flow-resistance relationship according to the at least one group of state parameters, then calculate a similarity between the actual flow-resistance relationship and each sample flow-resistance relationship in the preset database;
select a sample flow-resistance relationship which corresponds to a maximum similarity, if the maximum similarity is smaller than a first similarity threshold but greater than a second similarity threshold;
implement a calculation on the selected sample flow-resistance relationship according to the at least one group of state parameters, so as to obtain the target flow-resistance relationship.

In an embodiment, in the perfusion system, the processor is further configured to calculate an actual flow-resistance relationship according to the at least one group of state parameters, then calculate a similarity between the actual flow-resistance relationship and each sample flow-resistance relationship in the preset database; and generate an alarm indication if a maximum similarity is smaller than a second similarity threshold.

In an embodiment, in order to obtain an association relationship, the processor is further configured to:
obtain at least two groups of state parameters; and
fit to obtain a target flow-resistance relationship, which corresponds to a currently used endoscope system, according to the at least two groups of state parameters; wherein the target flow-resistance relationship is configured to indicate difference(s) between pressure value(s) measured by the pressure sensor and pressure value(s) at the outflow end of the endoscope system at different flow rates; and
use the target flow-resistance relationship as the association relationship.

In an embodiment, in order to obtain an association relationship, the processor is further configured to:
obtain a pressure value in the one group of state parameters;
define a relationship between the pressure value measured by the pressure sensor and the pressure value of the outflow end of the endoscope system as having a constant difference value, so as to obtain the association relationship; wherein the constant difference value is the pressure value in the one group of state parameters.

In an embodiment, in order to obtain an association relationship, the processor is further configured to:
obtain a preset initial flow-resistance relationship;
implement a calculation on the preset initial flow-resistance relationship according to the one group of state parameters, so as to obtain a target flow-resistance relationship, wherein the target flow-resistance relationship is configured to indicate difference(s) between pressure value(s) measured by the pressure sensor and pressure value(s) at the outflow end of the endoscope system at different flow rates; and
use the target flow-resistance relationship as the association relationship.

In an embodiment, the processor is further configured to adjust the pressure value of the outflow end of the endoscope system according to the association relationship; and/or to calculate the pressure value of the outflow end of the endoscope system according to the association relationship and to generate indication information.

In an embodiment, in order to adjust and/or calculate the pressure value of the outflow end of the endoscope system according to the association relationship, the processor is further configured to:
obtain a pump working parameter of the power pump mechanism, and calculate a resistance according to the pump working parameter of the power pump mechanism and the association relationship; and
adjust and/or calculate the pressure value of the outflow end of the endoscope system according to a pressure value of the pressure sensor and the calculated resistance.

In an embodiment, the pump working parameter of the power pump mechanism is a real-time pump working parameter; and the pressure value of the pressure sensor is a real-time pressure value.

In an embodiment, the processor is further configured to obtain real-time state parameters, and determine that the endoscope system has been replaced when the real-time state parameters do not match a current association relationship; wherein the state parameters include a pressure value which is obtained by the pressure sensor, and a flow value which is obtained by a pump working parameter of the power pump mechanism and/or by a flow sensor which is arranged at the perfusion pipeline.

In an embodiment, the pump working parameter includes a rotation speed of pump or a preset flow rate of medium.

In an embodiment, the perfusion system further includes one start key, which is configured to generate the start instruction after being triggered.

An embodiment provides a working method for a perfusion system, wherein the perfusion system includes a perfusion pipeline and a pressure sensor which is configured to measure a pressure value inside the perfusion pipeline; wherein the working method is applied to an endoscope system and includes:
obtaining, in response to an input of a start instruction, at least one group of state parameters of the perfusion pipeline, and obtaining an association relationship according to the state parameters; wherein the association relationship is configured to indicate a relationship between the pressure value measured by the pressure sensor and a pressure value of an outflow end of the endoscope system;
starting, in further response to the input of the start instruction, a surgical function which includes a perfusion function, an exhaust function, a flushing function and/or a cavity distending function.

An embodiment provides a working method for a perfusion system, wherein the perfusion system includes a perfusion pipeline and a pressure sensor which is configured to measure a pressure value inside the perfusion pipeline; wherein the working method is applied to an endoscope system and includes:
obtaining, in response to an input of a start instruction, at least one group of state parameters of the perfusion pipeline, and obtaining an association relationship according to the state parameters; wherein the association relationship is configured to indicate a relationship between the pressure value measured by the pressure sensor and a pressure value of an outflow end of the endoscope system;
starting, in further response to the input of the start instruction, a surgical function which is configured to enable a medium inside the perfusion pipeline to flow, so as to enter into a preset cavity of a living being through the perfusion pipeline and the endoscope system for performing surgery.

An embodiment provides a working method for a perfusion system, wherein the perfusion system includes a perfusion pipeline and a pressure sensor which is configured to measure a pressure value inside the perfusion pipeline; wherein the working method is applied to an endoscope system and includes:
pre-storing execution program(s) of a perfusion process of the perfusion system, wherein the perfusion process is at least divided into a first phase and a second phase;
enabling, in response to an input of a start instruction, the perfusion process to continuously enter into the first phase and the second phase;
wherein the start instruction is configured to start a surgical function which includes a perfusion function, an exhaust function, a flushing function and/or a cavity distending function; in the first phase, obtaining an association relationship, wherein the association relationship is configured to indicate a relationship between a pressure value measured by the pressure sensor and a pressure value of an outflow end of the endoscope system; in the second phase, using the association relationship to perform the surgical function.

An embodiment provides a working method for a perfusion system, wherein the perfusion system includes a perfusion pipeline and a pressure sensor which is configured to measure a pressure value inside the perfusion pipeline; wherein the working method is applied to an endoscope system and includes:
pre-storing execution program(s) of a perfusion process of the perfusion system, wherein the perfusion process is at least divided into a first phase and a second phase;
enabling, in response to an input of a start instruction, the perfusion process to continuously enter into the first phase and the second phase;
wherein the start instruction is configured to start a surgical function which includes a perfusion function, an exhaust function, a flushing function and/or a cavity distending function; in the first phase, obtaining at least one group of state parameters of the perfusion pipeline, wherein each group of state parameters include a pressure value which is obtained by the pressure sensor, and a flow value which is obtained by the pump working parameter of the power pump mechanism and/or by a flow sensor which is arranged at the perfusion pipeline; in the second phase, performing the surgical function according to the state parameters.

In an embodiment, performing the surgical function according to the state parameters, includes:
obtaining an association relationship according to the state parameters; and
using the association relationship to perform the surgical function.

An embodiment provides a working method for a perfusion system, wherein the perfusion system includes a perfusion pipeline and a pressure sensor which is configured to measure a pressure value inside the perfusion pipeline; wherein the working method is applied to an endoscope system and includes:
pre-storing execution program(s) of a perfusion process of the perfusion system, so as to perform a first function and a second function;
automatically performing, in response to an input of a start instruction, the first function and the second function; wherein performing the first function includes obtaining an association relationship, wherein the association relationship is configured to indicate a relationship between a pressure value measured by the pressure sensor and a pressure value of an outflow end of the endoscope system; performing the second function includes using the association relationship to perform a surgical function; wherein the surgical function includes a perfusion function, an exhaust function, a flushing function and/or a cavity distending function.

An embodiment provides a working method for a perfusion system, wherein the perfusion system includes a perfusion pipeline and a pressure sensor which is configured to measure a pressure value inside the perfusion pipeline; wherein the working method is applied to an endoscope system and includes:
obtaining, in response to an input of a start instruction, at least one group of state parameters of the perfusion pipeline, and obtaining an association relationship according to the state parameters; wherein the start instruction is configured to start a surgical function which includes a perfusion function, an exhaust function, a flushing function and/or a cavity distending function; wherein the association relationship is configured to indicate a relationship between a pressure value measured by the pressure sensor and a pressure value of an outflow end of the endoscope system;
wherein after the association relationship is obtained, re-obtaining at least one group of state parameters of the perfusion pipeline, and updating said association relationship according to the re-obtained state parameters, when the endoscope system is determined to have been replaced.

In an embodiment, obtaining the association relationship, includes:
obtaining one group of state parameters of the perfusion pipeline, and obtaining another group of state parameters of the perfusion pipeline after adjusting a pump working parameter of the power pump mechanism, wherein each group of state parameters include a pressure value which is obtained by the pressure sensor, and a flow value which is obtained by the pump working parameter of the power pump mechanism and/or by a flow sensor which is arranged at the perfusion pipeline; and
obtaining the association relationship according to the at least two groups of state parameters.

In an embodiment, obtaining one group of state parameters of the perfusion pipeline, and obtaining another group of state parameters of the perfusion pipeline after adjusting a pump working parameter of the power pump mechanism, includes:
adjusting a pump working parameter of the power pump mechanism, so as to enable a pressure value of the pressure sensor to be Fn, obtaining a current pump working parameter Rn of the power pump mechanism, and taking Fn and Rn as the one group of state parameters; and adjusting a pump working parameter of the power pump mechanism, so as to enable a pressure value of the pressure sensor to be Fn+1, obtaining a current pump working parameter Rn+1 of the power pump mechanism, and taking Fn+1 and Rn+1 as the another group of state parameters; or
adjusting a pump working parameter of the power pump mechanism to be R'n, so as to control the power pump mechanism to work, obtaining a pressure value F'n of the pressure sensor when said pump working parameter of the power pump mechanism is R'n, and taking F'n and R'n as the one group of state parameters; and adjusting a pump working parameter of the power pump mechanism to be R'n+1, so as to control the power pump mechanism to work, obtaining a pressure value F'n+1 of the pressure sensor when said pump working parameter of the power pump mechanism is R'n+1, and taking F'n+1 and R'n+1 as the another group of state parameters.

It can be understood that the above n and n+1 are subscripts indicating serial numbers.

In an embodiment, obtaining the association relationship, includes:
obtaining one group of state parameters of the perfusion pipeline, wherein the state parameters include a pressure value which is obtained by the pressure sensor, and a flow value which is obtained by the pump working parameter of the power pump mechanism and/or by a flow sensor which is arranged at the perfusion pipeline; and
obtaining the association relationship according to said one group of state parameters.

In an embodiment, obtaining the association relationship, includes:
selecting from a preset database, in which one or more sample flow-resistance relationships are preset, according to the at least one group of state parameters, one sample flow-resistance relationship as a target flow-resistance relationship; wherein the target flow-resistance relationship is configured to indicate difference(s) between pressure value(s) measured by the pressure sensor and pressure value(s) at the outflow end of the endoscope system at different flow rates; and
using the target flow-resistance relationship as the association relationship.

In an embodiment, selecting from a preset database, in which one or more sample flow-resistance relationships are preset, according to the at least one group of state parameters, one sample flow-resistance relationship as a target flow-resistance relationship, includes:
calculating an actual flow-resistance relationship according to the at least one group of state parameters, then calculating a similarity between the actual flow-resistance relationship and each sample flow-resistance relationship in the preset database;
selecting a sample flow-resistance relationship which corresponds to a maximum similarity as the target flow-resistance relationship, if the maximum similarity is greater than a first similarity threshold.

In an embodiment, selecting from a preset database, in which one or more sample flow-resistance relationships are preset, according to the at least one group of state parameters, one sample flow-resistance relationship as a target flow-resistance relationship, includes:
calculating an actual flow-resistance relationship according to the at least one group of state parameters, then calculating a similarity between the actual flow-resistance relationship and each sample flow-resistance relationship in the preset database;
selecting a sample flow-resistance relationship which corresponds to a maximum similarity, if the maximum similarity is smaller than a first similarity threshold but greater than a second similarity threshold;
implementing a calculation on the selected sample flow-resistance relationship according to the at least one group of state parameters, so as to obtain the target flow-resistance relationship.

In an embodiment, the working method further includes:
calculating an actual flow-resistance relationship according to the at least one group of state parameters, then calculating a similarity between the actual flow-resistance relationship and each sample flow-resistance relationship in the preset database; and
generating an alarm indication if a maximum similarity is smaller than a second similarity threshold.

In an embodiment, obtaining the association relationship, includes:
obtaining at least two groups of state parameters; and
fitting to obtain a target flow-resistance relationship, which corresponds to a currently used endoscope system, according to the at least two groups of state parameters; wherein the target flow-resistance relationship is configured to indicate difference(s) between pressure value(s) measured by the pressure sensor and pressure value(s) at the outflow end of the endoscope system at different flow rates; and
using the target flow-resistance relationship as the association relationship.

In an embodiment, obtaining the association relationship, includes:
obtaining a pressure value in the one group of state parameters;
defining a relationship between the pressure value measured by the pressure sensor and the pressure value of the outflow end of the endoscope system as having a constant difference value, so as to obtain the association relationship; wherein the constant difference value is the pressure value in the one group of state parameters.

In an embodiment, obtaining the association relationship, includes:
obtaining a preset initial flow-resistance relationship;
implementing a calculation on the preset initial flow-resistance relationship according to the one group of state parameters, so as to obtain a target flow-resistance relationship, wherein the target flow-resistance relationship is configured to indicate difference(s) between pressure value(s) measured by the pressure sensor and pressure value(s) at the outflow end of the endoscope system at different flow rates; and
using the target flow-resistance relationship as the association relationship.

In an embodiment, the working method further includes adjusting the pressure value of the outflow end of the endoscope system according to the association relationship; and/or calculating the pressure value of the outflow end of the endoscope system according to the association relationship and generating indication information.

In an embodiment, adjusting and/or calculating the pressure value of the outflow end of the endoscope system according to the association relationship, includes:
obtaining a pump working parameter of the power pump mechanism, and calculating a resistance according to the pump working parameter of the power pump mechanism and the association relationship; and
adjusting and/or calculating the pressure value of the outflow end of the endoscope system according to a pressure value of the pressure sensor and the calculated resistance.

In an embodiment, the pump working parameter of the power pump mechanism is a real-time pump working parameter; and the pressure value of the pressure sensor is a real-time pressure value.

In an embodiment, the working method further includes obtaining real-time state parameters, and determining that the endoscope system has been replaced when the real-time state parameters do not match a current association relationship; wherein the state parameters comprise a pressure value which is obtained by the pressure sensor, and a flow value which is obtained by a pump working parameter of the power pump mechanism of the perfusion system and/or by a flow sensor which is arranged at the perfusion pipeline.

In an embodiment, the pump working parameter includes a rotation speed of pump or a preset flow rate of medium.

In an embodiment, the perfusion system further includes one start key, which is configured to generate the start instruction after being triggered.

In an embodiment, an electronic device is provided, including:
a memory, which is configured to store program(s); and
a processor, which is configured to implement the method as described above in any embodiment of this disclosure by executing program(s) which is(are) stored in the memory.

In an embodiment, a computer-readable storage medium is provided, including program(s), which is(are) capable of being executed, so as to implement the method as described above in any embodiment of this disclosure.

According to the perfusion system and the working method for an endoscope system, the electronic device and the computer-readable storage medium implemented as above, a surgical function can be started, as well as an association relationship can be obtained through a start instruction, which is very convenient.

### BRIEF DESCRIPTION OF THE DRAWINGS

FIG. 1 is a diagram for flow-resistance relationships corresponding to different types of endoscope systems according to an embodiment.
FIG. 2 is a structural diagram for a perfusion system according to an embodiment.
FIG. 3 is a structural diagram for a perfusion system according to an embodiment.
FIG. 4 is a structural diagram for a perfusion system according to an embodiment.
FIG. 5 is a structural diagram for a perfusion system according to an embodiment.
FIG. 6 is a structural diagram for an endoscope system according to an embodiment.
FIG. 7 is a flow chart for a working method for a perfusion system according to an embodiment.
FIG. 8 is a flow chart for a working method for a perfusion system according to an embodiment.
FIG. 9 is a flow chart for a working method for a perfusion system according to an embodiment.
FIG. 10 is a flow chart for a working method for a perfusion system according to an embodiment.
FIG. 11 is a flowchart for obtaining an association relationship according to an embodiment.
FIG. 12 is a flowchart for obtaining an association relationship according to an embodiment.
FIG. 13 is a flowchart for obtaining an association relationship according to an embodiment.
FIG. 14 is a flowchart for obtaining an association relationship according to an embodiment.
FIG. 15 is a flowchart for obtaining an association relationship according to an embodiment.
FIG. 16 is a flowchart for obtaining an association relationship according to an embodiment.
FIG. 17 is a structural diagram for an electronic device according to an embodiment.

### DESCRIPTION OF THE PREFERRED EMBODIMENTS

This disclosure is further described in detail below through specific embodiments in combination with the accompanying drawings. In different embodiments, similar elements adopt associated similar reference numbers. In the following embodiments, many details are described in order to make this disclosure better understood. However, one skilled in the art can easily recognize that some of the features can be omitted in different cases, or can be replaced by other elements, materials and methods. In some cases, some operations related to this disclosure are not shown or described in the description, in order to avoid the core part of this disclosure being inundated by too many descriptions. For one skilled in the art, it is not necessary to describe these related operations in detail, as they can fully understand the relevant operations according to the description in this disclosure and the general technical knowledge in the art.

In addition, the features, operations, or characteristics described in the description may be combined in any appropriate manner to form various embodiments. At the same time, the steps or actions in the method description can also be exchanged or adjusted sequentially in a manner obvious to one skilled in the art. Therefore, the various sequences in the description and the drawings are only for the purpose of clearly describing a certain embodiment, and do not mean that they are necessary sequences. Unless it is otherwise specified that one of the sequences must be followed.

The terms "first", "second", etc. in the description are operable to distinguish different objects, rather than to describe a specific order. In addition, the terms "connection", and "linkage" mentioned in this disclosure include direct and indirect connection (linkage), unless otherwise specified.

Currently, a perfusion system and an endoscope system are two separate systems. The perfusion system and the endoscope system from different manufacturers can be combined and used. Under the current technical system, a pressure sensor is arranged at an end of the perfusion system, such as at a perfusion pipeline of the perfusion system, so that the pressure sensor cannot represent a true intracavitary pressure. One idea is to arrange the pressure sensor at an outlet end of a pipeline of the endoscope system, but as the pipeline of the endoscope system needs to be inserted into the cavity, this solution causes a complexity of the endoscope system, increases the cost a lot, and may cause incompatibility with other perfusion systems. It should be noted that the pressure sensor in this disclosure is configured to measure a pressure with a unit of force, such as Newton N, or a unit of intensity of pressure, such as Pascal Pa or millimetre of mercury mmHg, etc. The value obtained by the pressure sensor through measurement can be a resistance value, a capacitance value, a voltage value, a current value, or a pressure value, etc., which is configured to characterize a corresponding pressure.

The inventors studied measuring the pressure inside the cavity by using a pressure sensor at the end of the perfusion system. Specifically, since the pressure sensor is arranged at the perfusion system, it can only measure a pressure condition of the pipeline of the perfusion system. When the perfusion system starts working, a liquid inside the medium container, such as a liquid bag, enters into the endoscope system through the pipeline of the perfusion system. Therefore, a pipe wall of the perfusion system and the endoscope system produce a certain resistance to the liquid flowing inside the pipeline of the perfusion system. This resistance makes the pressure value measured by the pressure sensor at the end of the perfusion system to be higher than a pressure value at an outlet end of the pipeline of the endoscope system. The inventors discovered that the fluid resistance generated under a specific endoscope system has a specific relationship with a flow amount of the liquid at the perfusion system, which is referred to herein as a flow-resistance relationship (it may also be referred to as the flow-intensity of pressure relationship, i.e., the resistance here may be an intensity of pressure). Therefore, in some embodiments, the pressure value measured at the end of the perfusion system may be calculated or adjusted by using the flow amount of the pipeline and the corresponding functional relationship. In an example, a functional relationship between a flow amount of medium and a resistance of medium can be described by a quadratic function: P = A*Flow²+B*Flow.

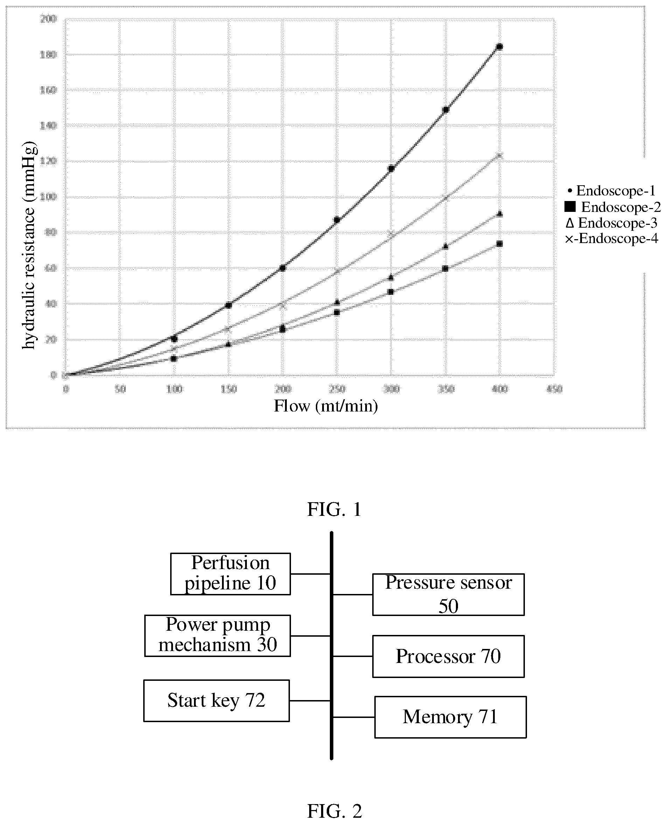

Wherein, P represents a resistance of medium, Flow represents a flow rate inside a pipeline, A and B are coefficients, which respectively represent a coefficient of a pressure or of an intensity of pressure of the above function. When a same perfusion system is used in combination with different endoscope systems, correction coefficients A and B mentioned above are different. FIG. 1 is an example, where endoscopes 1 to 4 (i.e., 4 different endoscope systems) correspond to four different curves. Of course, the functional relationship between the flow amount of medium and the resistance of medium can be described by a variety of functions, and the above is merely an example of describing this relationship by a quadratic function.

It can be seen that when the endoscope system paired with the perfusion system is changed, the functional relationship between the flow amount of medium and the resistance of medium may also change. There are many types of endoscope systems used in the clinical end, and the shapes of which are different. Therefore, different functional relationships need to be used for different endoscope systems. Therefore, one solution is that the user manually calibrates devices before use, that is, after the user assembles and docks the perfusion system with the endoscope system, before inserting the endoscope system into the body cavity of the patient, the functional relationship between a current flow rate of fluid and a resistance of fluid is firstly calibrated. Generally, a function type is fixed (for example, a quadratic function), so it is essentially to calculate the currently applicable coefficients A and B. After the calibration is completed, it can be guaranteed that the pressure value measured at the end of the perfusion system can obtain an accurate intracavitary pressure value.

In an embodiment, one calibration scheme or pairing scheme may be instruction in the product manual on how to manually calibrate and pair. In which, the perfusion system is firstly in respective connection with the medium container and the endoscope system, then a pump inside the perfusion system is manually started to exhaust, the calibration mode is then entered by manually selecting at the end of the perfusion system, and then the calibration is completed. During the calibration process, the user needs to hold the pump and pipeline of the perfusion system in hand all the time, and obtains data and performs calibrations by speeding up and down the pump at the end of the perfusion system. The whole process takes about 30 seconds. In addition to the fact that the entire calibration process is rather cumbersome, more importantly, since the user needs to manually select to enter into the calibration mode, the user may be not familiar with the machine and does not know that calibration is required before use, or the user may forget that calibration is required before use during use. As a result, in actual situations, the perfusion system and the endoscope system are often put into use without being calibrated, after being paired and docked.

Therefore, the above correction scheme is strongly complained by users in clinical use and is often ignored.

In another embodiment, the calibration and pairing scheme may be as follows. The manufacturer pre-sets flow-resistance relationships corresponding to a variety of endoscope systems at the end of the perfusion system, and the user only needs to select the type of endoscope system currently paired for use through an input tool before use. However, this scheme has poor scalability and is difficult to adapt to the various types of endoscope systems in the market. When the selected endoscope system does not match the endoscope system actually used, the accuracy of the intracavitary pressure calculation is affected.

In an embodiment of this disclosure, the pairing process is integrated into the preoperative preparation process and performed automatically, providing a senseless pairing. In another embodiment, the pairing process can be significantly shortened, and/or the pairing accuracy can be significantly improved, these can improve intelligence and safety of product.

Specifically, the inventors discovered that a natural operating process is as follows. The user docks one end of the pipeline of the perfusion system with the medium container, and docks the other end of the perfusion system with the pipeline of the endoscope system, and then starts the perfusion system. After the perfusion system is started, the medium inside the medium container flows through the pipeline of the perfusion system to the pipeline of the endoscope system, and flows out from the pipeline of the endoscope system. After the outflow situation of the liquid from the pipeline of the endoscope system is relatively stable (for example, the flow rate or flow amount is relatively stable), the pipeline of the endoscope system is inserted into the cavity to allow the liquid to enter into the cavity. The inventors considered proposing a solution to senseless integrate the pairing process into the above-mentioned operation flow.

A structure of a perfusion system is first described below.

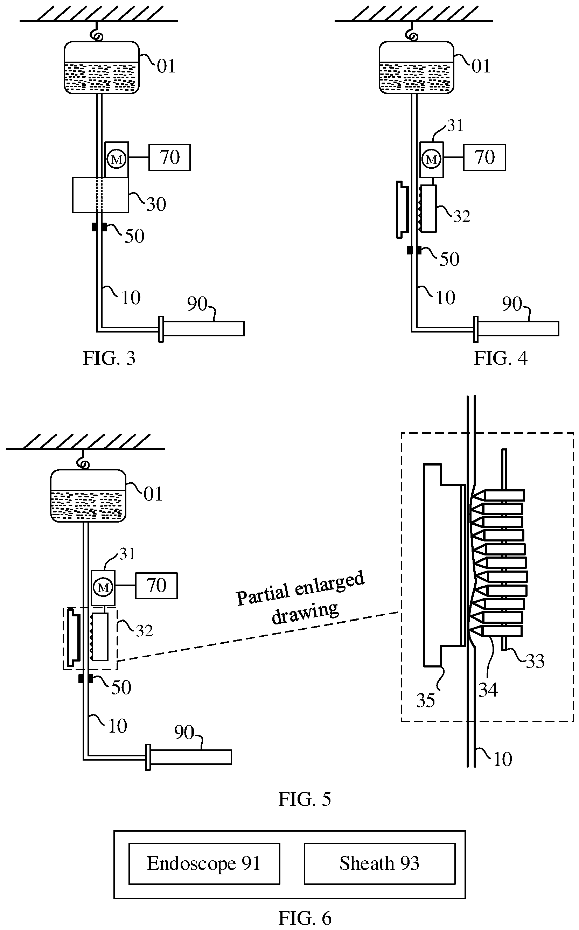

Please refer to FIG. 2 or FIG. 3. In an embodiment, the perfusion system includes a perfusion pipeline 10, a power pump mechanism 30, a pressure sensor 50 and a processor 70. In some embodiments, the perfusion system further includes a memory 71. In some embodiments, the perfusion system further includes a start key 72.

The perfusion system is applied to an endoscope system 90. For example, FIG. 3 is a schematic diagram in which a perfusion system and an endoscope system 90 are used in combination. The endoscope system 90 is configured to receive a medium (for example, a medium transported by the perfusion pipeline 10, which may be a liquid) and inject the medium into a body cavity to be observed through an outflow end of a pipeline of the endoscope system 90 itself to distend the cavity. A detailed description is given below.

One end of the perfusion pipeline 10 is connected with the medium container 01, and the other end of the perfusion pipeline 10 is connected with the endoscope system 90, so that the medium (such as liquid) inside the medium container 01 can flow into the endoscope system 90. The medium container 01 is a container for holding the medium, such as a liquid bag. The medium container 01 can be fixed by a wall hook or a bracket, etc., which is not limited here.

The power pump mechanism 30 is configured to drive the medium inside the medium container 01 to flow into the endoscope system 90 through the perfusion pipeline 10. In this way, through the driving of the power pump mechanism 30, the medium inside the perfusion pipeline 10 can flow in a desired direction and desired state (such as a desired flow rate or flow amount), that is, the medium inside the perfusion pipeline 10 can be driven to flow in a controllable manner. In some embodiments, the power pump mechanism 30 can work according to a pump working parameter, such as realizing a forward rotation and a reverse rotation, and such as working at a certain rotation speed. Therefore, in some embodiments, the pump working parameter include a rotation speed of pump. In other embodiments, the pump working parameter may further include a preset flow rate of medium. In some embodiments, by obtaining a preset flow rate of medium, the power pump mechanism 30 works at a corresponding rotation speed.

Referring to FIG. 4, in some embodiments, the power pump mechanism 30 may include a driving mechanism 31 and a squeezing mechanism 32. The squeezing mechanism 32 may be a peristaltic squeezing structure, a push squeezing structure, etc., which is not limited here. In some embodiments, the squeezing mechanism 32 is arranged at the perfusion pipeline 10 to squeeze the perfusion pipeline 10, so as to drive the medium inside the perfusion pipeline 10 to flow, and the squeezing mechanism 32 can drive the medium inside the perfusion pipeline 10 to flow in a controllable manner.

In some embodiments, referring to FIG. 5, the squeezing mechanism 32 includes a cam shaft 33, a group of pump plates 34 and a squeezing plate 35. When the cam shaft 33 rotates, the group of pump plates 34 on the cam shaft 33 performs linear reciprocating movement, that is, pump plates on the group of pump plates 34 perform linear reciprocating movement in sequence. The group of pump plates 34 cooperates with the squeezing plate 35 to squeeze and release an outer wall of the perfusion pipeline 10 sequentially and reciprocally, thus driving the medium inside the perfusion pipeline 10 to continuously flow in a directional manner.

The driving mechanism 31 is configured to drive the squeezing mechanism 32 to squeeze the perfusion pipeline 10 according to the pump working parameter. In some embodiments, the pump working parameter may be, for example, a rotation speed of pump or a preset flow rate of medium. In some embodiments, the driving mechanism 31 may include a power component, which may be an electromagnetic device that realizes electrical energy conversion or transmission based on a law of electromagnetic induction, such as a permanent magnet (PM) motor, a reactive (VR) motor, and a hybrid (HB) motor, etc. The power component is configured to drive the squeezing mechanism 32 to move, thereby squeezing the perfusion pipeline 10. Furthermore, the driving mechanism 31 may also include one or more force transmission/conversion components to cooperate with the power component. Specifically, when the power component moves, the one or more force transmission/conversion components are configured to drive the squeezing mechanism 32 to move, thereby squeezing the perfusion pipeline 10, so as to drive the medium inside the perfusion pipeline 10 to flow. In some embodiments, the one or more force transmission/conversion components may include, for example, gear(s), transmission shaft(s), screw rod(s), nut(s), or slider(s).

A driving process is as follows. The processor 70 transmits instructions, such as a speed or a position (a pump working parameter), so as to enable the driving mechanism 31 to work at a specified speed and direction of rotation. During its rotation process, the driving mechanism 31 drives the cam shaft 33 in the squeezing mechanism 32 to rotate, wherein the cam shaft 33 is connected with the driving mechanism 31. During the rotation of the cam shaft 33, the group of pump plates 34 at the cam shaft 33 performs linear reciprocating movement, that is, the pump plates in the group of pump plates 34 perform linear reciprocating movement in sequence. The group of pump plates 34 cooperates with the squeezing plate 35 to squeeze and release the outer wall of the perfusion pipeline 10 sequentially and reciprocally, thus driving the medium inside the perfusion pipeline 10 to continuously flow in a directional manner. Wherein a speed reduction mechanism may be provided between the driving mechanism 31 and the cam shaft 33 in the squeezing mechanism 32 to ensure that the rotation speed of the group of pump plates 34 is stable and uniform.

The above is some description of the power pump mechanism 30 .

The pressure sensor 50 is configured to measure a pressure value inside the perfusion pipeline 10. The pressure value obtained by the pressure sensor 50 may be a resistance value, a capacitance value, a voltage value, a current value, or an intensity of pressure value, etc. These values may be used to characterize a pressure value at a corresponding location of the perfusion pipeline 10. The unit of the pressure value may be a unit of force, such as Newton N, or a unit of intensity of pressure, such as Pascal Pa or millimetre of mercury mmHg, etc.

In some embodiments, the pressure sensor 50 may be arranged near a location where the perfusion pipeline 10 is acted upon by the power pump mechanism 30 .

The above are some instructions for the perfusion system.

The endoscope system 90 is described below.

In some embodiments, the endoscope system 90 is capable of receiving a medium (e.g., liquid) which is inputted from the perfusion pipeline 10, and then further transmitting the medium to a preset cavity of organism, so as to distend the cavity, etc. In some embodiments, the endoscope system 90 obtains an image signal in the cavity and transmits the image signal out, for example, to its own display screen or other display screens for displaying, so as to enable the user to observe. In some embodiments, in order to obtain a clearer image, the endoscope system 90 also has a light source and is capable of transmitting light into the cavity through a related optical path structure (e.g., optical fibre), so as to illuminate the cavity.

Please refer to FIG. 6. In some embodiments, the endoscope system 90 includes an endoscope 91 and a sheath 93. In some embodiments, the endoscope 91 has a flow channel, which can cooperate with the perfusion pipeline 10 of the perfusion system, such that the medium inside the perfusion pipeline 10 can enter into the preset cavity of the organism through the flow channel of the endoscope 91. In some embodiments, the sheath 93 includes a tubular sheath body, an interior of which is capable of passing through the endoscope 91. In some embodiments, the endoscope 91 may be a ureteroscope or a nephroscope, etc.

The above is some description of the endoscope system 90 .

In some embodiments, in the perfusion system of this disclosure, its processor 70 is configured to obtain, in response to an input of a start instruction, at least one group of state parameters of the perfusion pipeline 10, and to obtain an association relationship according to the state parameters; wherein the start instruction is configured to start a surgical function, and the association relationship is configured to indicate a relationship between a pressure value measured by the pressure sensor 50 and a pressure value of an outflow end of the endoscope system 90.

In some embodiments, the memory 71 is configured to store execution program(s) of a perfusion process of the perfusion system, and the perfusion process is divided into at least a first phase and a second phase. The processor 70 is configured to enable, in response to an input of a start instruction, the perfusion process to continuously enter into the above-mentioned first phase and the above-mentioned second phase; wherein the start instruction is configured to start a surgical function. In the first phase, the processor 70 obtains an association relationship, wherein the association relationship is configured to indicate a relationship between a pressure value measured by the pressure sensor 50 and a pressure value of the outflow end of the endoscope system 90; and in the second phase, the processor 70 is configured to use the association relationship to perform the surgical function.

In some embodiments, the memory 71 is configured to store execution program(s) of a perfusion process of the perfusion system, and the perfusion process is divided into at least a first phase and a second phase. The processor 70 is configured to enable, in response to an input of a start instruction, the perfusion process to continuously enter into the above-mentioned first phase and the above-mentioned second phase; wherein the start instruction is configured to start a surgical function. In the first phase, the processor 70 obtains at least one group of state parameters of the perfusion pipeline 10; in the second phase, the processor 70 is configured to perform a surgical function according to the state parameters. In some embodiments, the processor 70 obtains an association relationship according to the state parameters, and uses the association relationship to perform the surgical function, wherein the association relationship is configured to indicate a relationship between a pressure value measured by the pressure sensor 50 and a pressure value of the outflow end of the endoscope system 90.

In some embodiments, the memory 71 is configured to store execution program(s) of a perfusion process of the perfusion system, so as to perform a first function and a second function. The processor 70 is configured to automatically execute, in response to an input of a start instruction, the first function and the second function; wherein, in order to implement the first function, the processor 70 is further configured to obtain an association relationship, wherein the association relationship is configured to indicate a relationship between a pressure value measured by the pressure sensor 50 and a pressure value of an outflow end of the endoscope system 90; in order to implement the second function, the processor 70 is further configured to use the association relationship to perform a surgical function.

In some embodiments, the processor 70 is configured to obtain, in response to an input of a start instruction, at least one group of state parameters of the perfusion pipeline 10, and configured to obtain an association relationship according to the state parameters; wherein the start instruction is configured to start a surgical function; the association relationship is configured to indicate a relationship between a pressure value measured by the pressure sensor 50 and a pressure value of the outflow end of the endoscope system 90. After obtaining the association relationship, the processor 70 is further configured to re-obtain at least one group of state parameters of the perfusion pipeline and update the association relationship according to the re-obtained state parameters, when determining that the endoscope system 90 is replaced. In some embodiments, whether the endoscope system 90 is replaced can be determined in the following manner. The processor 70 is configured to obtain real-time state parameters, and when it is determined that the real-time state parameters do not match a current association relationship, the endoscope system 90 is determined to have been replaced.

In some embodiments, the surgical function is configured to enable the medium inside the perfusion pipeline 10 to flow, so as to enter into a preset cavity of a living being through the perfusion pipeline 10 and the endoscope system 90 for performing surgery; wherein the surgical function includes, for example, a perfusion function, an exhaust function, a flushing function and/or a cavity distending function.

In some embodiments, each group of state parameters include a pressure value which is obtained by the pressure sensor 30, and a flow value which is directly or indirectly obtained by a pump working parameter of the power pump mechanism 30 and/or by a flow sensor which is arranged at the perfusion pipeline 10.

In some embodiments, a start key 72 is configured to generate a start instruction after being triggered.

The following describes how the processor obtains the association relationship.

In some embodiments, the processor 70 obtains one group of state parameters of the perfusion pipeline 10, and obtains another group of state parameters of the perfusion pipeline 10 after adjusting a pump working parameter of the power pump mechanism 30. The processor 70 obtains the association relationship according to the at least two groups of state parameters.

In some embodiments, the processor 70 adjusts a pump working parameter of the power pump mechanism 30, so as to enable a pressure value of the pressure sensor to be Fn, obtains a current pump working parameter Rn of the power pump mechanism 30, and takes Fn and Rn as the one group of state parameters; and the processor 70 adjusts a pump working parameter of the power pump mechanism 30, so as to enable a pressure value of the pressure sensor to be changed to Fn+1, obtains a current pump working parameter Rn+1 of the power pump mechanism 30, and takes Fn+1 and Rn+1 as the another group of state parameters. For example, the processor 70 controls the power pump mechanism 30 to work, so as to enable a pressure value or a measured value of the pressure sensor 50 to change in sequence to N values, namely F1, F2, ..., Fn. In this way, the pipeline of the endoscope system 90 goes through N states in sequence, such as a state which corresponds to the pressure value F1 of the pressure sensor 30, a state which corresponds to the pressure value F2 of the pressure sensor 30, ..., until a state which corresponds to the pressure value Fn of the pressure sensor 30, and the processor 70 obtains a pump working parameter of the power pump mechanism 30 in each state.

In some embodiments, the processor 70 adjusts a pump working parameter of the power pump mechanism 30 to R'n, so as to control the power pump mechanism 30 to work, and obtains a pressure value F'n of the pressure sensor 50 when said pump working parameter of the power pump mechanism 30 is R'n, and takes F'n and R'n as the one group of state parameters; and the processor 70 adjusts a pump working parameter of the power pump mechanism 30 to be R'n+1, so as to control the power pump mechanism 30 to work, and obtains a pressure value F'n+1 of the pressure sensor 50 when said pump working parameter of the power pump mechanism 30 is R'n+1, and takes F'n+1 and R'n+1 as the another group of state parameters. For example, the processor 70 controls a pump working parameter to be R'1, R'2, ..., R'n in sequence, so as to enable the power pump mechanism 30 to work in sequence according to the pump working parameter R'1, R'2, ..., R'n. In this way, the pipeline of the endoscope system 90 goes through N states in sequence, such as a state which corresponds to the pump working parameter R'1 of the power pump mechanism 30, a state which corresponds to the pump working parameter R'2 of the power pump mechanism 30, until a state which corresponds to the pump working parameter R'n of the power pump mechanism 30. The processor 70 obtains a pressure value of the pressure sensor 30 in each state.

In some embodiments, the processor 70 obtains one group of state parameters of the perfusion pipeline 10, and the processor 70 obtains the association relationship according to said one group of state parameters.

The following describes how to obtain the association relationship according to the state parameters.

In some embodiments, the processor 70 selects from a preset database, in which one or more sample flow-resistance relationships are preset, according to at least one group of state parameters (such as one group, two groups or more than two groups), one sample flow-resistance relationship as a target flow-resistance relationship; wherein the target flow-resistance relationship is configured to indicate difference(s) between pressure value(s) measured by the pressure sensor 30 and pressure value(s) at an outflow end of the endoscope system 90 at different flow rates.

In some specific embodiments, the processor 70 calculates an actual flow-resistance relationship according to the at least one group of state parameters, and then calculates a similarity between the actual flow-resistance relationship and each sample flow-resistance relationship in the above-mentioned preset database. In some embodiments, if a maximum similarity is greater than a first similarity threshold, the processor 70 selects a sample flow-resistance relationship which corresponds to the maximum similarity as the target flow-resistance relationship. In some embodiments, if the maximum similarity is smaller than a first similarity threshold but greater than a second similarity threshold, the processor 70 selects a sample flow-resistance relationship which corresponds to the maximum similarity, and implements a calculation on the selected sample flow-resistance relationship according to the at least one group of state parameters, so as to obtain the target flow-resistance relationship. In some embodiments, if a maximum similarity is smaller than a second similarity threshold, the processor 70 generates an alarm indication.

In some embodiments, the processor 70 obtains at least two groups of state parameters, and fits to obtain a target flow-resistance relationship, which corresponds to a currently used endoscope system, according to the at least two groups of state parameters; wherein the target flow-resistance relationship is configured to indicate difference(s) between pressure value(s) measured by the pressure sensor 30 and pressure value(s) at an outflow end of the endoscope system 90 at different flow rates; and the processor 70 uses the target flow-resistance relationship as the association relationship.

In some embodiments, the processor 70 obtains a pressure value in one group of state parameters; the processor 70 defines a relationship between a pressure value measured by the pressure sensor 30 and a pressure value of the outflow end of the endoscope system 90 as having a constant difference value, so as to obtain the association relationship; wherein the constant difference value is the pressure value in the one group of state parameters.

In some embodiments, the processor 70 obtains a preset initial flow-resistance relationship; implements a calculation on the preset initial flow-resistance relationship according to the one group of state parameters, so as to obtain a target flow-resistance relationship, wherein the target flow-resistance relationship is configured to indicate difference(s) between pressure value(s) measured by the pressure sensor 30 and pressure value(s) at an outflow end of the endoscope system 90 at different flow rates; and the processor 70 uses the target flow-resistance relationship as the association relationship.

In some embodiments, the processor 70 adjusts a pressure value of an outflow end of the endoscope system 90 according to the association relationship.

In some embodiments, the processor 70 calculates a pressure value of an outflow end of the endoscope system according to the association relationship and generates indication information.

In some embodiments, the processor 70 obtains a pump working parameter of the power pump mechanism 30 (e.g., a real-time pump working parameter), and calculates a resistance according to the pump working parameter of the power pump mechanism 30 and the association relationship; the processor 70 adjusts and/or calculates the pressure value of the outflow end of the endoscope system based on a pressure value of the pressure sensor (e.g., a real-time pressure value) and the calculated resistance.

The surgical function is performed by using the association relationship as follows. The processor 70 adjusts the pressure value of the outflow end of the endoscope system 90 according to the association relationship, or the processor 70 calculates the pressure value of the outflow end of the endoscope system according to the association relationship and generates indication information, such as display information for displaying the pressure value of the outflow end of the endoscope system.

In some embodiments of this disclosure, a working method for a perfusion system is also disclosed. The working method is capable of being applied to the endoscope system 90. The working method for a perfusion system is described below.

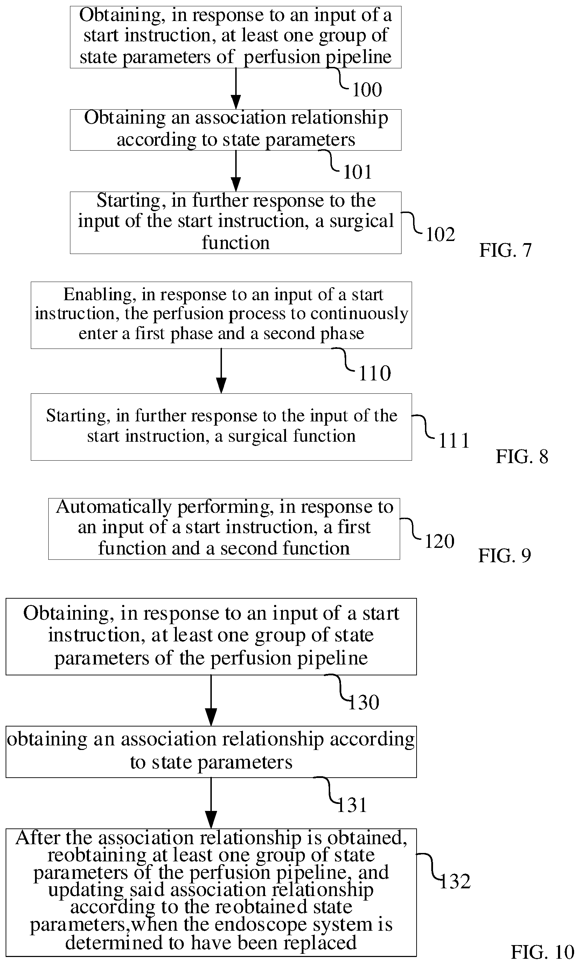

Referring to FIG. 7, a working method for a perfusion system of some embodiments includes following steps:
step 100, obtaining, in response to an input of a start instruction, at least one group of state parameters of the perfusion pipeline 10;
step 101, obtaining an association relationship according to the state parameters; wherein the association relationship is configured to indicate a relationship between a pressure value measured by the pressure sensor and a pressure value of an outflow end of the endoscope system; and
step 102, starting, in further response to the input of the start instruction, a surgical function.

Referring to FIG. 8, a working method for a perfusion system of some embodiments pre-stores execution program(s) of a perfusion process of the perfusion system, and the perfusion process is divided into at least a first phase and a second phase. The working method includes following steps:
step 110, enabling, in response to an input of a start instruction, the perfusion process to continuously enter into the first phase and the second phase; and
step 111, starting, in further response to the input of the start instruction, a surgical function.

In some embodiments, in the first phase, an association relationship is obtained, wherein the association relationship is configured to indicate relationship between a pressure value measured by the pressure sensor 50 and a pressure value of the outflow end of the endoscope system 90; and in the second phase, the association relationship is used to perform the surgical function.

In some embodiments, in the first phase, at least one group of state parameters of the perfusion pipeline 10 is obtained; and in the second phase, the surgical function is performed according to the state parameters. In some embodiments, an association relationship is obtained according to the state parameters, and the surgical function is performed by using the association relationship, wherein the association relationship is configured to indicate a relationship between a pressure value measured by the pressure sensor 50 and a pressure value of the outflow end of the endoscope system 90 .

Referring to FIG. 9, a working method for a perfusion system of some embodiments pre-stores execution program(s) of a perfusion process of the perfusion system, so as to at least perform a first function and a second function. The working method for a perfusion system includes a following step:
step 120, automatically performing, in response to an input of a start instruction, the first function and the second function. In some embodiments, performing the first function includes obtaining an association relationship, wherein the association relationship is configured to indicate a relationship between a pressure value measured by the pressure sensor 50 and a pressure value of an outflow end of the endoscope system 90; performing the second function includes using the association relationship to perform a surgical function.

Referring to FIG. 10, a working method for a perfusion system of some embodiments includes following steps:
step 130, obtaining, in response to an input of a start instruction, at least one group of state parameters of the perfusion pipeline 10, wherein the start instruction is configured to start a surgical function;
step 131, obtaining an association relationship according to the state parameters; wherein the association relationship is configured to indicate a relationship between a pressure value measured by the pressure sensor 50 and a pressure value of the outflow end of the endoscope system 90;
step 132, after the association relationship is obtained, re-obtaining at least one group of state parameters of the perfusion pipeline, and updating said association relationship according to the re-obtained state parameters, when the endoscope system 90 is determined to have been replaced. In some embodiments, step 132 determines whether the endoscope system 90 has been replaced by obtaining real-time state parameters, and determining that the endoscope system 90 is replaced when the real-time state parameters do not match the current association relationship.

In some embodiments, the surgical function is configured to enable the medium inside the perfusion pipeline 10 to flow, so as to enter into a preset cavity of a living being through the perfusion pipeline 10 and the endoscope system 90 for performing surgery; wherein the surgical function includes, for example, a perfusion function, an exhaust function, a flushing function and/or a cavity distending function.

In some embodiments, each group of state parameters include a pressure value which is obtained by the pressure sensor 30, and a flow value which is directly or indirectly obtained by a pump working parameter of the power pump mechanism 30 and/or by a flow sensor which is arranged at the perfusion pipeline 10.

In some embodiments, a start key 72 is configured to generate a start instruction after being triggered.

The following describes how the processor obtains the association relationship.

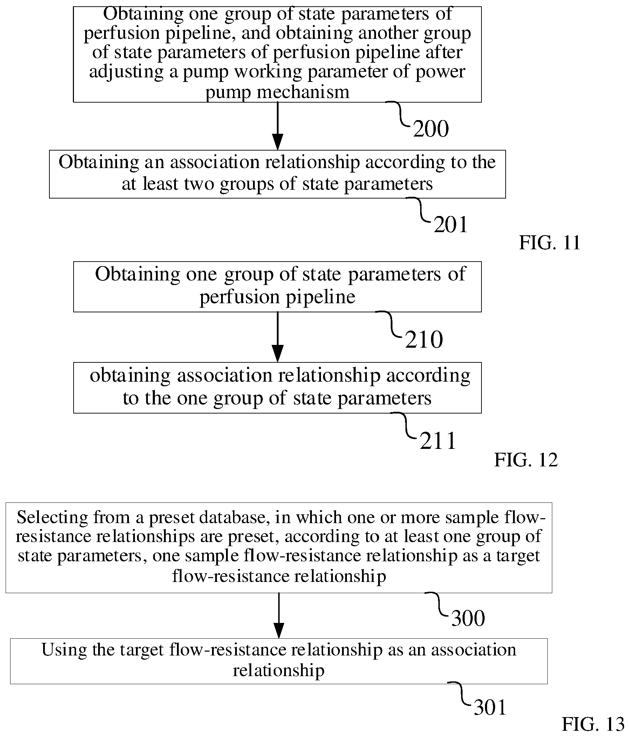

Referring to FIG. 11, in some embodiments, obtaining an association relationship includes following steps.

In step 200, obtaining one group of state parameters of the perfusion pipeline 10, and obtaining another group of state parameters of the perfusion pipeline 10 after adjusting a pump working parameter of the power pump mechanism 30.

In some embodiments, the step 200 adjusts a pump working parameter of the power pump mechanism 30, so as to enable a pressure value of the pressure sensor to be Fn, obtains a current pump working parameter Rn of the power pump mechanism 30, and takes Fn and Rn as the one group of state parameters; and the step 200 adjusts a pump working parameter of the power pump mechanism 30, so as to enable a pressure value of the pressure sensor to be changed to Fn+1, obtains a current pump working parameter Rn+1 of the power pump mechanism 30, and takes Fn+1 and Rn+1 as the another group of state parameters. For example, the step 200 controls the power pump mechanism 30 to work, so as to enable a pressure value or a measured value of the pressure sensor 50 to change in sequence to N values, namely F1, F2, ..., Fn. In this way, the pipeline of the endoscope system 90 goes through N states in sequence, such as a state which corresponds to the pressure value F1 of the pressure sensor 30, a state which corresponds to the pressure value F2 of the pressure sensor 30, ..., until a state which corresponds to the pressure value Fn of the pressure sensor 30, and the step 200 obtains a pump working parameter of the power pump mechanism 30 in each state.

In some embodiments, the step 200 adjusts a pump working parameter of the power pump mechanism 30 to R'n, so as to control the power pump mechanism 30 to work, and obtains a pressure value F'n of the pressure sensor 50 when said pump working parameter of the power pump mechanism 30 is R'n, and takes F'n and R'n as the one group of state parameters; and the step 200 adjusts a pump working parameter of the power pump mechanism 30 to be R'n+1, so as to control the power pump mechanism 30 to work, and obtains a pressure value F'n+1 of the pressure sensor 50 when said pump working parameter of the power pump mechanism 30 is R'n+1, and takes F'n+1 and R'n+1 as the another group of state parameters. For example, the step 200 controls the pump working parameter to be R'1, R'2, ..., R'n in sequence, so as to enable the power pump mechanism 30 to work in sequence according to the pump working parameter R'1, R'2, ..., R'n. In this way, the pipeline of the endoscope system 90 goes through N states in sequence, such as a state which corresponds to the pump working parameter R'1 of the power pump mechanism 30, a state which corresponds to the pump working parameter R'2 of the power pump mechanism 30, until a state which corresponds to the pump working parameter R'n of the power pump mechanism 30. The step 200 obtains a pressure value of the pressure sensor 30 in each state.

In step 201, obtaining an association relationship according to the at least two groups of state parameters.

Referring to FIG. 12, in some embodiments, obtaining an association relationship includes following steps:
step 210, obtaining one group of state parameters of the perfusion pipeline 10;
Step 211, obtaining the association relationship according to said one group of state parameters in step 210.

The following describes how to obtain the association relationship according to the state parameters.

Referring to FIG. 13, in some embodiments, obtaining an association relationship includes following steps.
step 300, selecting from a preset database, in which one or more sample flow-resistance relationships are preset, according to at least one group of state parameters (such as one group, two groups or more than two groups), one sample flow-resistance relationship as a target flow-resistance relationship; wherein the target flow-resistance relationship is configured to indicate difference(s) between pressure value(s) measured by the pressure sensor 30 and pressure value(s) at an outflow end of the endoscope system 90 at different flow rates.

In some specific embodiments, the step 301 calculates an actual flow-resistance relationship according to the at least one group of state parameters, and then calculates a similarity between the actual flow-resistance relationship and each sample flow-resistance relationship in the above-mentioned preset database. In some embodiments, if the maximum similarity is greater than a first similarity threshold, the step 301 selects a sample flow-resistance relationship which corresponds to the maximum similarity as the target flow-resistance relationship. In some embodiments, if the maximum similarity is smaller than a first similarity threshold but greater than a second similarity threshold, the step 301 selects a sample flow-resistance relationship which corresponds to the maximum similarity, and implements a calculation on the selected sample flow-resistance relationship according to the at least one group of state parameters, so as to obtain the target flow-resistance relationship.

Step 301, using the target flow-resistance relationship as an association relationship.

In some embodiments, the working method may further include a step of generating an alarm indication, if a maximum similarity is smaller than a second similarity threshold.

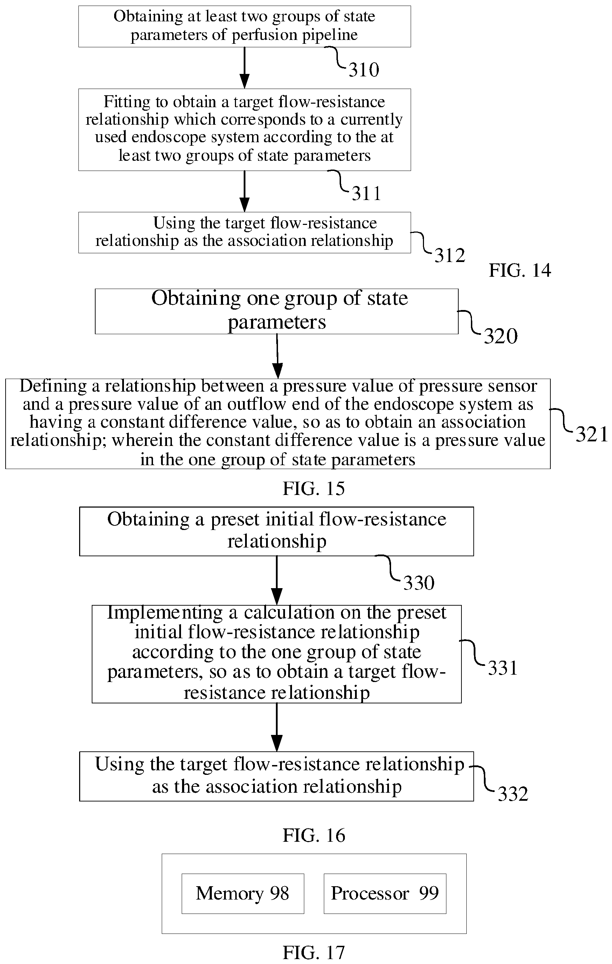

Referring to FIG. 14, in some embodiments, obtaining an association relationship includes following steps:
step 310, obtaining at least two groups of state parameters of the perfusion pipeline 10;
Step 311, fitting to obtain a target flow-resistance relationship which corresponds to a currently used endoscope system 90 according to the at least two groups of state parameters in the step 310; wherein the target flow-resistance relationship is configured to indicate difference(s) between pressure value(s) measured by the pressure sensor 30 and pressure value(s) at an outflow end of the endoscope system 90 at different flow rates; and
step 312, using the target flow-resistance relationship as the association relationship.

Referring to FIG. 15, in some embodiments, obtaining an association relationship includes following steps:
S320, obtaining a pressure value in one group of state parameters;
S321, defining a relationship between a pressure value measured by the pressure sensor 30 and a pressure value of an outflow end of the endoscope system 90 as having a constant difference value, so as to obtain the association relationship; wherein the constant difference value is the pressure value in the one group of state parameters.

Referring to FIG. 16, in some embodiments, obtaining an association relationship includes following steps:
Step 330, obtaining a preset initial flow-resistance relationship; and
Step 331, implementing a calculation on the preset initial flow-resistance relationship according to one group of state parameters, so as to obtain a target flow-resistance relationship, wherein the target flow-resistance relationship is configured to indicate difference(s) between pressure value(s) measured by the pressure sensor 30 and pressure value(s) at an outflow end of the endoscope system 90 at different flow rates;
Step 332, using the target flow-resistance relationship as the association relationship.

In some embodiments, the working method further includes: adjusting the pressure value of the outflow end of the endoscope system 90 according to the association relationship; and/or calculating the pressure value of the outflow end of the endoscope system according to the association relationship, and generating indication information; such as, obtaining a pump working parameter of the power pump mechanism 30 (such as a real-time pump working parameter), and calculating a resistance according to the pump working parameter of the power pump mechanism 30 and the association relationship; and adjusting and/or calculating the pressure value of the outflow end of the endoscope system according to the pressure value of the pressure sensor (such as a real-time pressure value) and the calculated resistance.

It should be noted that, preforming a surgical function by using the association relationship can be adjusting the pressure value of the outflow end of the endoscope system 90 according to the association relationship, or calculating the pressure value of the outflow end of the endoscope system according to the association relationship and generating indication information, such as display information for displaying the pressure value of the outflow end of the endoscope system.

Referring to FIG. 17, some embodiments further disclose an electronic device, which includes a memory 98 and a processor 99. The memory 98 stores a program(s), and the processor 99 is configured to implement the method as described above in any embodiment of this disclosure by executing program(s) which is(are) stored in the memory 98.

All or part of the above embodiments can be implemented by software, hardware, firmware, or any combination thereof. In addition, as understood by those skilled in the art, the principles herein may be reflected in a computer program product on a computer-readable storage medium that is pre-installed with computer-readable program codes. Any tangible, non-transitory computer-readable storage medium can be used, including magnetic storage devices (hard disks, floppy disks, etc.), optical storage devices (CD-ROM, DVD, Blu Ray disks, etc.), flash memory, and/or the like. These computer program instructions can be loaded onto a general-purpose computer, a dedicated computer, or other programmable data processing device to form a machine, so that these instructions, which are executed on a computer or other programmable data processing apparatus, can generate an apparatus that implements a specified function. These computer program instructions can also be stored in a computer-readable memory that can instruct a computer or other programmable data processing device to operate in a specific manner, so that the instructions stored in the computer-readable memory can form a manufactured product, including an implementation apparatus that implements a specified function. The computer program instructions can also be loaded onto a computer or other programmable data processing device, so that a series of operating steps are performed on the computer or other programmable device to produce a computer-implemented process, so that the instructions executed on a computer or other programmable data processing device can provide steps for implementing specified functions.

Although the principles herein have been shown in various embodiments, many modifications of structures, arrangements, proportions, elements, materials, and components particularly suitable for the specific environmental and operational requirements may be used without departing from the principles and scope of the present disclosure. The above modifications and other changes or amendments will be included in the scope of this disclosure.

The foregoing specific description has been described with reference to various embodiments. However, those skilled in the art will recognize that various modifications and changes can be made without departing from the scope of the present disclosure. Therefore, consideration of the present disclosure will be in an illustrative rather than a restrictive sense, and all such modifications will be included within the scope thereof. Also, the advantages of various embodiments, other advantages, and the solutions to problems have been described above. However, the benefits, advantages, solutions to problems, and any elements that can produce these, or solutions that make them more explicit, should not be interpreted as critical, necessary, or essential. The term "including", and any other variants thereof used herein are non-exclusive, so that the process, method, document, or device that includes a list of elements includes not only these elements, but also other elements that are not explicitly listed or do not belong to the process, method, system, document, or device. Furthermore, the term "coupling" and any other variations thereof used herein refer to physical connection, electrical connection, magnetic connection, optical connection, communicational connection, functional connection, and/or any other connection.

It should be noted that a person of ordinary skill in the art could also make several variations and improvements without departing from the concept of this disclosure, and these variations and improvements would all fall within the scope of protection of this disclosure. Therefore, the protection scope of this disclosure should be in accordance with the appended claims.

## Claims

1. A perfusion system for an endoscope system, **characterized in that**, comprising:
a perfusion pipeline, wherein one end of the perfusion pipeline is connected with a medium container, and the other end of the perfusion pipeline is connected with the endoscope system;
a power pump mechanism, which is configured to drive a medium inside the medium container to flow into the endoscope system through the perfusion pipeline;
a pressure sensor, which is configured to measure a pressure value inside the perfusion pipeline; and
a processor, which is configured to obtain, in response to an input of a start instruction, one group of state parameters of the perfusion pipeline, and to obtain another group of state parameters of the perfusion pipeline after adjusting a pump working parameter of the power pump mechanism, wherein each group of state parameters comprise a pressure value which is obtained by the pressure sensor, and a flow value which is obtained by the pump working parameter of the power pump mechanism and/or by a flow sensor which is arranged at the perfusion pipeline; wherein the start instruction is configured to start a surgical function which comprises a perfusion function, an exhaust function, a flushing function and/or a cavity distending function;
the processor is further configured to calculate an actual flow-resistance relationship according to the at least two groups of state parameters, calculate a similarity between the actual flow-resistance relationship and each sample flow-resistance relationship in a preset database, and obtain a target flow-resistance relationship according to the similarity; or the processor is further configured to fit to obtain a target flow-resistance relationship, which corresponds to a currently used endoscope system, according to the at least two groups of state parameters;
the processor is further configured to obtain a real-time pump working parameter of the power pump mechanism, and calculate a resistance according to the real-time pump working parameter of the power pump mechanism and the target flow-resistance relationship; and to adjust and/or calculate a real-time pressure value of an outflow end of the endoscope system according to a real-time pressure value of the pressure sensor and the calculated resistance.

2. A perfusion system for an endoscope system, **characterized in that**, comprising:
a perfusion pipeline, wherein one end of the perfusion pipeline is connected with a medium container, and the other end of the perfusion pipeline is connected with the endoscope system;
a power pump mechanism, which is configured to drive a medium inside the medium container to flow into the endoscope system through the perfusion pipeline;
a pressure sensor, which is configured to measure a pressure value inside the perfusion pipeline; and
a processor, which is configured to obtain, in response to an input of a start instruction, at least one group of state parameters of the perfusion pipeline, and to obtain an association relationship according to the state parameters; wherein the start instruction is configured to start a surgical function which comprises a perfusion function, an exhaust function, a flushing function and/or a cavity distending function; wherein the association relationship is configured to indicate a relationship between a pressure value measured by the pressure sensor and a pressure value of an outflow end of the endoscope system.

3. A perfusion system for an endoscope system, **characterized in that**, comprising:
a perfusion pipeline, wherein one end of the perfusion pipeline is connected with a medium container, and the other end of the perfusion pipeline is connected with the endoscope system;
a power pump mechanism, which is configured to drive a medium inside the medium container to flow into the endoscope system through the perfusion pipeline;
a pressure sensor, which is configured to measure a pressure value inside the perfusion pipeline; and
a processor, which is configured to obtain, in response to an input of a start instruction, at least one group of state parameters of the perfusion pipeline, and to obtain an association relationship according to the state parameters; wherein the start instruction is configured to start a surgical function, which is configured to enable the medium inside the perfusion pipeline to flow, so as to enter into a preset cavity of a living being through the perfusion pipeline and the endoscope system for performing surgery; wherein the association relationship is configured to indicate a relationship between a pressure value measured by the pressure sensor and a pressure value of an outflow end of the endoscope system.

4. A perfusion system for an endoscope system, **characterized in that**, comprising:
a perfusion pipeline, wherein one end of the perfusion pipeline is connected with a medium container, and the other end of the perfusion pipeline is connected with the endoscope system;
a power pump mechanism, which is configured to drive a medium inside the medium container to flow into the endoscope system through the perfusion pipeline;
a pressure sensor, which is configured to measure a pressure value inside the perfusion pipeline; and
a memory, which is configured to store execution program(s) of a perfusion process of the perfusion system, wherein the perfusion process is at least divided into a first phase and a second phase;
a processor, which is configured to enable, in response to an input of a start instruction, the perfusion process to continuously enter into the first phase and the second phase;
wherein the start instruction is configured to start a surgical function which comprises a perfusion function, an exhaust function, a flushing function and/or a cavity distending function; in the first phase, the processor is further configured to obtain an association relationship, wherein the association relationship is configured to indicate a relationship between a pressure value measured by the pressure sensor and a pressure value of an outflow end of the endoscope system; in the second phase, the processor is further configured to use the association relationship to perform the surgical function.

5. A perfusion system for an endoscope system, **characterized in that**, comprising:
a perfusion pipeline, wherein one end of the perfusion pipeline is connected with a medium container, and the other end of the perfusion pipeline is connected with the endoscope system;
a power pump mechanism, which is configured to drive a medium inside the medium container to flow into the endoscope system through the perfusion pipeline;
a pressure sensor, which is configured to measure a pressure value inside the perfusion pipeline; and
a memory, which is configured to store execution program(s) of a perfusion process of the perfusion system, wherein the perfusion process is at least divided into a first phase and a second phase;
a processor, which is configured to enable, in response to an input of a start instruction, the perfusion process to continuously enter into the first phase and the second phase;
wherein the start instruction is configured to start a surgical function which comprises a perfusion function, an exhaust function, a flushing function and/or a cavity distending function; in the first phase, the processor is further configured to obtain at least one group of state parameters of the perfusion pipeline, wherein each group of state parameters comprise a pressure value which is obtained by the pressure sensor, and a flow value which is obtained by a pump working parameter of the power pump mechanism and/or by a flow sensor which is arranged at the perfusion pipeline; in the second phase, the processor is further configured to perform the surgical function according to the state parameters.

6. The perfusion system for an endoscope system according to claim 5, **characterized in that**, in order to perform the surgical function according to the state parameters, the processor is further configured to:
obtain an association relationship according to the state parameters; wherein the association relationship is configured to indicate a relationship between a pressure value measured by the pressure sensor and a pressure value of an outflow end of the endoscope system; and
use the association relationship to perform the surgical function.

7. A perfusion system for an endoscope system, **characterized in that**, comprising:
a perfusion pipeline, wherein one end of the perfusion pipeline is connected with a medium container, and the other end of the perfusion pipeline is connected with the endoscope system;
a power pump mechanism, which is configured to drive a medium inside the medium container to flow into the endoscope system through the perfusion pipeline;
a pressure sensor, which is configured to measure a pressure value inside the perfusion pipeline; and
a memory, which is configured to store execution program(s) of a perfusion process of the perfusion system, so as to perform a first function and a second function;
a processor, which is configured to automatically perform, in response to an input of a start instruction, the first function and the second function; wherein in order to perform the first function, the processor is further configured to obtain an association relationship, wherein the association relationship is configured to indicate a relationship between a pressure value measured by the pressure sensor and a pressure value of an outflow end of the endoscope system; in order to perform the second function, the processor is further configured to use the association relationship to perform a surgical function;
wherein the surgical function comprises a perfusion function, an exhaust function, a flushing function and/or a cavity distending function.

8. A perfusion system for an endoscope system, **characterized in that**, comprising:
a perfusion pipeline, wherein one end of the perfusion pipeline is connected with a medium container, and the other end of the perfusion pipeline is connected with the endoscope system;
a power pump mechanism, which is configured to drive a medium inside the medium container to flow into the endoscope system through the perfusion pipeline;
a pressure sensor, which is configured to measure a pressure value inside the perfusion pipeline; and
a processor, which is configured to obtain, in response to an input of a start instruction, at least one group of state parameters of the perfusion pipeline, and to obtain an association relationship according to the state parameters; wherein the start instruction is configured to start a surgical function which comprises a perfusion function, an exhaust function, a flushing function and/or a cavity distending function; wherein the association relationship is configured to indicate a relationship between a pressure value measured by the pressure sensor and a pressure value of an outflow end of the endoscope system;
wherein after obtaining the association relationship, the processor is further configured to re-obtain at least one group of state parameters of the perfusion pipeline and update the association relationship according to the re-obtained state parameters, when determining that the endoscope system has been replaced.

9. The perfusion system for an endoscope system according to claim 2, 3, 4, 6, 7 or 8, **characterized in that**, in order to obtain an association relationship, the processor is further configured to:
obtain one group of state parameters of the perfusion pipeline, and obtain another group of state parameters of the perfusion pipeline after adjusting a pump working parameter of the power pump mechanism, wherein each group of state parameters comprise a pressure value which is obtained by the pressure sensor, and a flow value which is obtained by the pump working parameter of the power pump mechanism and/or by a flow sensor which is arranged at the perfusion pipeline; and
obtain the association relationship according to the at least two groups of state parameters.

10. The perfusion system for an endoscope system according to claim 9, **characterized in that**, in order to obtain one group of state parameters of the perfusion pipeline, and obtain another group of state parameters of the perfusion pipeline after adjusting a pump working parameter of the power pump mechanism, the processor is further configured to:
adjust the pump working parameter of the power pump mechanism, so as to enable the pressure value of the pressure sensor to be Fn, obtain a current pump working parameter Rn of the power pump mechanism, and take Fn and Rn as the one group of state parameters; and adjust the pump working parameter of the power pump mechanism, so as to enable the pressure value of the pressure sensor to be Fn+1, obtain a current pump working parameter Rn+1 of the power pump mechanism, and take Fn+1 and Rn+1 as the another group of state parameters; or
adjust the pump working parameter of the power pump mechanism to be R'n, so as to control the power pump mechanism to work, obtain the pressure value F'n of the pressure sensor when said pump working parameter of the power pump mechanism is R'n, and take F'n and R'n as the one group of state parameters; and adjust the pump working parameter of the power pump mechanism to be R'n+1, so as to control the power pump mechanism to work, obtain the pressure value F'n+1 of the pressure sensor when said pump working parameter of the power pump mechanism is R'n+1, and take F'n+1 and R'n+1 as the another group of state parameters.

11. The perfusion system for an endoscope system according to claim 2, 3, 4, 6, 7 or 8, **characterized in that**, in order to obtain an association relationship, the processor is further configured to:
obtain one group of state parameters of the perfusion pipeline, wherein the state parameters comprise a pressure value which is obtained by the pressure sensor, and a flow value which is obtained by a pump working parameter of the power pump mechanism and/or by a flow sensor which is arranged at the perfusion pipeline; and
obtain the association relationship according to said one group of state parameters.

12. The perfusion system for an endoscope system according to claim 2, 3, 4, 6, 7, 8, 9, or 11, **characterized in that**, in order to obtain an association relationship, the processor is further configured to:
select from a preset database, in which one or more sample flow-resistance relationships are preset, one sample flow-resistance relationship as a target flow-resistance relationship, according to at least one group of state parameters; wherein the target flow-resistance relationship is configured to indicate difference(s) between pressure value(s) measured by the pressure sensor and pressure value(s) at the outflow end of the endoscope system at different flow rates; and
use the target flow-resistance relationship as the association relationship.

13. The perfusion system for an endoscope system according to claim 12, **characterized in that**, in order to select from a preset database, in which one or more sample flow-resistance relationships are preset, one sample flow-resistance relationship as a target flow-resistance relationship, according to at least one group of state parameters, the processor is further configured to:
calculate an actual flow-resistance relationship according to the at least one group of state parameters, then calculate a similarity between the actual flow-resistance relationship and each sample flow-resistance relationship in the preset database; and
select a sample flow-resistance relationship which corresponds to a maximum similarity as the target flow-resistance relationship, if the maximum similarity is greater than a first similarity threshold.

14. The perfusion system for an endoscope system according to claim 13, **characterized in that**, in order to select from a preset database, in which one or more sample flow-resistance relationships are preset, one sample flow-resistance relationship as a target flow-resistance relationship, according to at least one group of state parameters, the processor is further configured to:
calculate an actual flow-resistance relationship according to the at least one group of state parameters, then calculate a similarity between the actual flow-resistance relationship and each sample flow-resistance relationship in the preset database;
select a sample flow-resistance relationship which corresponds to a maximum similarity, if the maximum similarity is smaller than a first similarity threshold but greater than a second similarity threshold; and
implement a calculation on the selected sample flow-resistance relationship according to the at least one group of state parameters, so as to obtain the target flow-resistance relationship.

15. The perfusion system for an endoscope system according to claim 13, **characterized in that**, the processor is further configured to calculate an actual flow-resistance relationship according to the at least one group of state parameters, then calculate a similarity between the actual flow-resistance relationship and each sample flow-resistance relationship in the preset database; and generate an alarm indication if a maximum similarity is smaller than a second similarity threshold.

16. The perfusion system for an endoscope system according to claim 9, **characterized in that**, in order to obtain an association relationship, the processor is further configured to:
obtain the at least two groups of state parameters; and
fit to obtain a target flow-resistance relationship, which corresponds to a currently used endoscope system, according to the at least two groups of state parameters; wherein the target flow-resistance relationship is configured to indicate difference(s) between pressure value(s) measured by the pressure sensor and pressure value(s) at the outflow end of the endoscope system at different flow rates; and
use the target flow-resistance relationship as the association relationship.

17. The perfusion system for an endoscope system according to claim 11, **characterized in that**, in order to obtain an association relationship, the processor is further configured to:
obtain a pressure value in the one group of state parameters;
define a relationship between the pressure value measured by the pressure sensor and the pressure value of the outflow end of the endoscope system as having a constant difference value, so as to obtain the association relationship; wherein the constant difference value is the pressure value in the one group of state parameters.

18. The perfusion system for an endoscope system according to claim 11, **characterized in that**, in order to obtain an association relationship, the processor is further configured to:
obtain a preset initial flow-resistance relationship;
implement a calculation on the preset initial flow-resistance relationship according to the one group of state parameters, so as to obtain a target flow-resistance relationship, wherein the target flow-resistance relationship is configured to indicate difference(s) between pressure value(s) measured by the pressure sensor and pressure value(s) at the outflow end of the endoscope system at different flow rates; and
use the target flow-resistance relationship as the association relationship.

19. The perfusion system for an endoscope system according to any one of claims 2-4 or claims 6-18, **characterized in that**, the processor is further configured to adjust the pressure value of the outflow end of the endoscope system according to the association relationship; and/or to calculate the pressure value of the outflow end of the endoscope system according to the association relationship and to generate indication information.

20. The perfusion system for an endoscope system according to claim 19, **characterized in that**, in order to adjust and/or calculate the pressure value of the outflow end of the endoscope system according to the association relationship, the processor is further configured to:
obtain a pump working parameter of the power pump mechanism, and calculate a resistance according to the pump working parameter of the power pump mechanism and the association relationship; and
adjust and/or calculate the pressure value of the outflow end of the endoscope system according to a pressure value of the pressure sensor and the calculated resistance.

21. The perfusion system for an endoscope system according to claim 20, **characterized in that**, the pump working parameter of the power pump mechanism is a real-time pump working parameter; and the pressure value of the pressure sensor is a real-time pressure value.

22. The perfusion system for an endoscope system according to claim 8, **characterized in that**, the processor is further configured to obtain real-time state parameters, and determine that the endoscope system has been replaced when the real-time state parameters do not match a current association relationship; wherein the state parameters comprise a pressure value which is obtained by the pressure sensor, and a flow value which is obtained by a pump working parameter of the power pump mechanism and/or by a flow sensor which is arranged at the perfusion pipeline.

23. The perfusion system for an endoscope system according to claim 5, 9, 10, 11, 20, 21 or 22, **characterized in that**, the pump working parameter comprises a rotation speed of the pump or a preset flow rate of the medium.

24. The perfusion system for an endoscope system according to any one of claims 1-5 or claims 7-8, **characterized in that**, further comprising one start key, which is configured to generate the start instruction after being triggered.

25. A working method for a perfusion system, wherein the perfusion system comprises a perfusion pipeline and a pressure sensor which is configured to measure a pressure value inside the perfusion pipeline; wherein the working method is applied to an endoscope system, **characterized in that**, the working method comprises:
obtaining, in response to an input of a start instruction, at least one group of state parameters of the perfusion pipeline, and obtaining an association relationship according to the state parameters; wherein the association relationship is configured to indicate a relationship between a pressure value measured by the pressure sensor and a pressure value of an outflow end of the endoscope system;
starting, in further response to the input of the start instruction, a surgical function which comprises a perfusion function, an exhaust function, a flushing function and/or a cavity distending function.

26. A working method for a perfusion system, wherein the perfusion system comprises a perfusion pipeline and a pressure sensor which is configured to measure a pressure value inside the perfusion pipeline; wherein the working method is applied to an endoscope system, **characterized in that**, the working method comprises:
obtaining, in response to an input of a start instruction, at least one group of state parameters of the perfusion pipeline, and obtaining an association relationship according to the state parameters; wherein the association relationship is configured to indicate a relationship between a pressure value measured by the pressure sensor and a pressure value of an outflow end of the endoscope system;
starting, in further response to the input of the start instruction, a surgical function which is configured to enable a medium inside the perfusion pipeline to flow, so as to enter into a preset cavity of a living being through the perfusion pipeline and the endoscope system for performing surgery.

27. A working method for a perfusion system, wherein the perfusion system comprises a perfusion pipeline and a pressure sensor which is configured to measure a pressure value inside the perfusion pipeline; wherein the working method is applied to an endoscope system, **characterized in that**, the working method comprises:
pre-storing execution program(s) of a perfusion process of the perfusion system, wherein the perfusion process is at least divided into a first phase and a second phase;
enabling, in response to an input of a start instruction, the perfusion process to continuously enter into the first phase and the second phase;
wherein the start instruction is configured to start a surgical function which comprises a perfusion function, an exhaust function, a flushing function and/or a cavity distending function; in the first phase, obtaining an association relationship, wherein the association relationship is configured to indicate a relationship between a pressure value measured by the pressure sensor and a pressure value of an outflow end of the endoscope system; in the second phase, using the association relationship to perform the surgical function.

28. A working method for a perfusion system, wherein the perfusion system comprises a perfusion pipeline and a pressure sensor which is configured to measure a pressure value inside the perfusion pipeline; wherein the working method is applied to an endoscope system, **characterized in that**, the working method comprises:
pre-storing execution program(s) of a perfusion process of the perfusion system, wherein the perfusion process is at least divided into a first phase and a second phase;
enabling, in response to an input of a start instruction, the perfusion process to continuously enter into the first phase and the second phase;
wherein the start instruction is configured to start a surgical function which comprises a perfusion function, an exhaust function, a flushing function and/or a cavity distending function; in the first phase, obtaining at least one group of state parameters of the perfusion pipeline, wherein each group of state parameters comprise a pressure value which is obtained by the pressure sensor, and a flow value which is obtained by a pump working parameter of a power pump mechanism of the perfusion system and/or by a flow sensor which is arranged at the perfusion pipeline; in the second phase, performing the surgical function according to the state parameters.

29. The working method according to claim 28, **characterized in that**, performing the surgical function according to the state parameters, comprises:
obtaining an association relationship according to the state parameters; and
using the association relationship to perform the surgical function.

30. A working method for a perfusion system, wherein the perfusion system comprises a perfusion pipeline and a pressure sensor which is configured to measure a pressure value inside the perfusion pipeline; wherein the working method is applied to an endoscope system, **characterized in that**, the working method comprises:
pre-storing execution program(s) of a perfusion process of the perfusion system, so as to perform a first function and a second function;
automatically performing, in response to an input of a start instruction, the first function and the second function; wherein performing the first function comprises obtaining an association relationship, wherein the association relationship is configured to indicate a relationship between a pressure value measured by the pressure sensor and a pressure value of an outflow end of the endoscope system; performing the second function comprises using the association relationship to perform a surgical function; wherein the surgical function comprises a perfusion function, an exhaust function, a flushing function and/or a cavity distending function.

31. A working method for a perfusion system, wherein the perfusion system comprises a perfusion pipeline and a pressure sensor which is configured to measure a pressure value inside the perfusion pipeline; wherein the working method is applied to an endoscope system, **characterized in that**, the working method comprises:
obtaining, in response to an input of a start instruction, at least one group of state parameters of the perfusion pipeline, and obtaining an association relationship according to the state parameters; wherein the start instruction is configured to start a surgical function which comprises a perfusion function, an exhaust function, a flushing function and/or a cavity distending function; wherein the association relationship is configured to indicate a relationship between a pressure value measured by the pressure sensor and a pressure value of an outflow end of the endoscope system;
wherein after the association relationship is obtained, re-obtaining at least one group of state parameters of the perfusion pipeline, and updating said association relationship according to the re-obtained state parameters, when the endoscope system is determined to have been replaced.

32. The working method according to claim 25, 26, 27, 29, 30 or 31, **characterized in that**, obtaining an association relationship, comprises:
obtaining one group of state parameters of the perfusion pipeline, and obtaining another group of state parameters of the perfusion pipeline after adjusting a pump working parameter of a power pump mechanism of the perfusion system, wherein each group of state parameters comprise a pressure value which is obtained by the pressure sensor, and a flow value which is obtained by the pump working parameter of the power pump mechanism of the perfusion system and/or by a flow sensor which is arranged at the perfusion pipeline; and
obtaining the association relationship according to the at least two groups of state parameters.

33. The working method according to claim 32, **characterized in that**, obtaining one group of state parameters of the perfusion pipeline, and obtaining another group of state parameters of the perfusion pipeline after adjusting a pump working parameter of a power pump mechanism of the perfusion system, comprises:
adjusting the pump working parameter of the power pump mechanism, so as to enable the pressure value of the pressure sensor to be Fn, obtaining a current pump working parameter Rn of the power pump mechanism, and taking Fn and Rn as the one group of state parameters; and adjusting the pump working parameter of the power pump mechanism, so as to enable the pressure value of the pressure sensor to be Fn+1, obtaining a current pump working parameter Rn+1 of the power pump mechanism, and taking Fn+1 and Rn+1 as the another group of state parameters; or
adjusting the pump working parameter of the power pump mechanism to be R'n, so as to control the power pump mechanism to work, obtaining the pressure value F'n of the pressure sensor when said pump working parameter of the power pump mechanism is R'n, and taking F'n and R'n as the one group of state parameters; and adjusting the pump working parameter of the power pump mechanism to be R'n+1, so as to control the power pump mechanism to work, obtaining the pressure value F'n+1 of the pressure sensor when said pump working parameter of the power pump mechanism is R'n+1, and taking F'n+1 and R'n+ 1 as the another group of state parameters.

34. The working method according to claim 25, 26, 27, 29, 30 or 31, **characterized in that**, obtaining an association relationship, comprises:
obtaining one group of state parameters of the perfusion pipeline, wherein the state parameters comprise a pressure value which is obtained by the pressure sensor, and a flow value which is obtained by a pump working parameter of a power pump mechanism of the perfusion system and/or by a flow sensor which is arranged at the perfusion pipeline; and
obtaining the association relationship according to said one group of state parameters.

35. The working method according to claim 25, 26, 27, 29, 30, 31, 32 or 34, **characterized in that**, obtaining an association relationship, comprises:
selecting from a preset database, in which one or more sample flow-resistance relationships are preset, one sample flow-resistance relationship as a target flow-resistance relationship, according to at least one group of state parameters; wherein the target flow-resistance relationship is configured to indicate difference(s) between pressure value(s) measured by the pressure sensor and pressure value(s) at the outflow end of the endoscope system at different flow rates; and
using the target flow-resistance relationship as the association relationship.

36. The working method according to claim 35, **characterized in that**, selecting from a preset database, in which one or more sample flow-resistance relationships are preset, one sample flow-resistance relationship as a target flow-resistance relationship, according to at least one group of state parameters, comprises:
calculating an actual flow-resistance relationship according to the at least one group of state parameters, then calculating a similarity between the actual flow-resistance relationship and each sample flow-resistance relationship in the preset database;
selecting a sample flow-resistance relationship which corresponds to a maximum similarity as the target flow-resistance relationship, if the maximum similarity is greater than a first similarity threshold.

37. The working method according to claim 36, **characterized in that**, selecting from a preset database, in which one or more sample flow-resistance relationships are preset, one sample flow-resistance relationship as a target flow-resistance relationship, according to at least one group of state parameters, comprises:
calculating an actual flow-resistance relationship according to the at least one group of state parameters, then calculating a similarity between the actual flow-resistance relationship and each sample flow-resistance relationship in the preset database;
selecting a sample flow-resistance relationship which corresponds to a maximum similarity, if the maximum similarity is smaller than a first similarity threshold but greater than a second similarity threshold;
implementing a calculation on the selected sample flow-resistance relationship according to the at least one group of state parameters, so as to obtain the target flow-resistance relationship.

38. The working method according to claim 36, **characterized in that**, further comprising:
calculating an actual flow-resistance relationship according to the at least one group of state parameters, then calculating a similarity between the actual flow-resistance relationship and each sample flow-resistance relationship in the preset database; and
generating an alarm indication if a maximum similarity is smaller than a second similarity threshold.

39. The working method according to claim 32, **characterized in that**, obtaining an association relationship, comprises:
obtaining the at least two groups of state parameters; and
fitting to obtain a target flow-resistance relationship, which corresponds to a currently used endoscope system, according to the at least two groups of state parameters; wherein the target flow-resistance relationship is configured to indicate difference(s) between pressure value(s) measured by the pressure sensor and pressure value(s) at the outflow end of the endoscope system at different flow rates; and
using the target flow-resistance relationship as the association relationship.

40. The working method according to claim 34, **characterized in that**, obtaining an association relationship, further comprises:
obtaining a pressure value in the one group of state parameters;
defining a relationship between the pressure value measured by the pressure sensor and the pressure value of the outflow end of the endoscope system as having a constant difference value, so as to obtain the association relationship; wherein the constant difference value is the pressure value in the one group of state parameters.

41. The working method according to claim 34, **characterized in that**, obtaining an association relationship, further comprises:
obtaining a preset initial flow-resistance relationship;
implementing a calculation on the preset initial flow-resistance relationship according to the one group of state parameters, so as to obtain a target flow-resistance relationship, wherein the target flow-resistance relationship is configured to indicate difference(s) between pressure value(s) measured by the pressure sensor and pressure value(s) at the outflow end of the endoscope system at different flow rates; and
using the target flow-resistance relationship as the association relationship.

42. The working method according to any one of claims 25-27 or claims 29-41, **characterized in that**, further comprising:
adjusting the pressure value of the outflow end of the endoscope system according to the association relationship; and/or calculating the pressure value of the outflow end of the endoscope system according to the association relationship and generating indication information.

43. The working method according to claim 42, **characterized in that**, adjusting and/or calculating the pressure value of the outflow end of the endoscope system according to the association relationship, comprises:
obtaining a pump working parameter of a power pump mechanism, and calculating a resistance according to the pump working parameter of the power pump mechanism and the association relationship; and
adjusting and/or calculating the pressure value of the outflow end of the endoscope system according to a pressure value of the pressure sensor and the calculated resistance.

44. The working method according to claim 43, **characterized in that**, the pump working parameter of the power pump mechanism is a real-time pump working parameter; and the pressure value of the pressure sensor is a real-time pressure value.

45. The working method according to claim 31, **characterized in that**, further comprising obtaining real-time state parameters, and determining that the endoscope system has been replaced when the real-time state parameters do not match a current association relationship; wherein the state parameters comprise a pressure value which is obtained by the pressure sensor, and a flow value which is obtained by a pump working parameter of a power pump mechanism of the perfusion system and/or by a flow sensor which is arranged at the perfusion pipeline.

46. The working method according to claim 28, 32, 33, 34, 43 or 44, **characterized in that**, the pump working parameter comprises a rotation speed of the pump or a preset flow rate of the medium.

47. The working method according to any one of claims 25-30, **characterized in that**, the perfusion system further comprises one start key, which is configured to generate the start instruction after being triggered.

48. An electronic device, **characterized in that**, comprising:
a memory, which is configured to store program(s); and
a processor, which is configured to implement the method according to any one of claims 25-47 by executing program(s) which is(are) stored in the memory.

49. A computer-readable storage medium, **characterized in that**, comprising program(s), which is(are) capable of being executed, so as to implement the method according to any one of claims 25-48.
